(19) Europäisches Patentamt
European Patent Office
Office européen des brevets

(11) EP 4 585 223 A2

(12) EUROPEAN PATENT APPLICATION

(43) Date of publication:
16.07.2025 Bulletin 2025/29

(21) Application number: 25165433.1

(22) Date of filing: 14.01.2021

(51) International Patent Classification (IPC):
A61K 39/00 (2006.01)

(52) Cooperative Patent Classification (CPC):
(C-Sets available)
A61K 31/573; A61K 31/12; A61K 31/513;
A61K 35/34; A61K 38/13; A61P 11/00        (Cont.)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 15.01.2020   US 202062961636 P
31.03.2020   US 202063003264 P

(62) Document number(s) of the earlier application(s) in
accordance with Art. 76 EPC:
21741877.1 / 4 090 349

(71) Applicant: The Regents of the University of
California
Oakland, CA 94607 (US)

(72) Inventors:
• SCHREPFER, Sonja
California, 94607 (US)

• DEUSE, Tobias
California, 94607 (US)

(74) Representative: Bassil, Nicholas Charles et al
Kilburn & Strode LLP
Lacon London
84 Theobalds Road
London WC1X 8NL (GB)

Remarks:
•The complete document including Reference
Table(s) and the Sequence Listing(s) can be
downloaded from the EPO website
•This application was filed on 21-03-2025 as a
divisional application to the application mentioned
under INID code 62.
•Claims filed after the date of filing of the divisional
application (Rule 68(4) EPC).

(54) TRANSPLANTED CELL PROTECTION VIA INHIBITION OF POLYMORPHONUCLEAR CELLS

(57) The invention provides, for the first time, strategies to inhibit the killing of transplanted cells by activated polymorphonuclear cells (PMNs) of the recipient. Multiple different modes for PMN inhibition are provided and one or more agents effectively utilized every mode of action. The combination of two or more of those agents with different modes of action synergistically improved the efficacy of PMN inhibition without exerting toxic side effects on the survival of the target cells. The cells may be pluripotent cells, including hypoimmune pluripotent cells (HIP), ABO blood type O Rhesus Factor negative HIP cells (HIPO-), or derivatives thereof. The cells may also be alpha 1 antitrypsin (A1AT) secreting cells.

EP 4 585 223 A2

(52) Cooperative Patent Classification (CPC): (Cont.)

C-Sets
**A61K 31/513, A61K 2300/00;**
**A61K 38/13, A61K 2300/00**

**Description**

I. **CROSS REFERENCE TO RELATED APPLICATIONS**

This application claims the benefit of U.S. Provisional Application No. 62/961,636 filed on January 15, 2020 and U.S. Provisional Application No. 63/003,264 filed March 31, 2020.

II. **FIELD OF THE INVENTION**

The invention relates to regenerative cell therapy. In some embodiments, the regenerative cell therapy comprises transplanting cells or cell lines into patients in need thereof. In some embodiments, the cells or cell lines comprise regenerative cell therapies having a reduced sensitivity to the cell transplant recipient's immune system that kills the cells during the window of time shortly after transplantation. In some embodiments, the regenerative cell therapy is used in the treatment of injured organs and tissue. In some embodiments, the regenerative cell therapy of the invention utilizes chimeric antigen receptor (CAR) cells, endothelial cells, fibrocytes, dopaminergic neurons, pancreatic islet cells, hepatocytes, cardiomyocytes, or retinal pigment endothelium cells are used for treating diseases or rehabilitating damaged tissues. In some embodiments, the regenerative cell therapy is used to treat alpha-1 antitrypsin deficiency.

III. **BACKGROUND OF THE INVENTION**

Regenerative cell therapy is an important potential treatment for regenerating injured organs and tissue. With the low availability of organs for transplantation and the accompanying lengthy wait, the possibility of regenerating tissue by transplanting readily available cell lines into patients is understandably appealing. Regenerative cell therapy has shown promising initial results for rehabilitating damaged tissues after transplantation in animal models (e.g. after myocardial infarction). The propensity for the transplant recipient's immune system to reject allogeneic material, however, greatly reduces the potential efficacy of therapeutics and diminishes the possible positive effects surrounding such treatments.

Autologous induced pluripotent stem cells (iPSCs) theoretically constitute an unlimited cell source for patient-specific cell-based organ repair strategies. Their generation, however, poses technical and manufacturing challenges and is a lengthy process that conceptually prevents any acute treatment modalities. Allogeneic iPSC-based therapies or embryonic stem cell-based therapies are easier from a manufacturing standpoint and allow the generation of well-screened, standardized, high-quality cell products. Because of their allogeneic origin, however, such cell products would undergo rejection. With the reduction or elimination of the cells' antigenicity, universally-acceptable cell products could be produced. Because pluripotent stem cells can be differentiated into any cell type of the three germ layers, the potential application of stem cell therapy is wide-ranging. Differentiation can be performed *ex vivo* or *in vivo* by transplanting progenitor cells that continue to differentiate and mature in the organ environment of the implantation site. *Ex vivo* differentiation allows researchers or clinicians to closely monitor the procedure and ensures that the proper population of cells is generated prior to transplantation.

In most cases, however, undifferentiated pluripotent stem cells are avoided in clinical transplant therapies due to their propensity to form teratomas. Rather, such therapies tend to use differentiated cells (e.g. stem cell-derived cardiomyocytes transplanted into the myocardium of patients suffering from heart failure). Clinical applications of such pluripotent cells or tissues would benefit from a "safety feature" that controls the growth and survival of cells after their transplantation.

The art seeks stem cells capable of producing cells that are used to regenerate or replace diseased or deficient cells. Pluripotent stem cells (PSCs) may be used as a source for producing differentiated cell products because they rapidly propagate and differentiate into many possible cell types. The family of PSCs includes several members generated via different techniques and possessing distinct immunogenic features. Patient compatibility with engineered cells or tissues derived from PSCs determines the risk of immune rejection and the requirement for immunosuppression.

Embryonic stem cells (ESCs) isolated from the inner cell mass of blastocysts exhibit histocompatibility antigens that are mismatches with virtually all possible recipients. This immunological barrier cannot be solved by human leukocyte antigen (HLA)-typed banks of ESCs because even HLA-matched PSC grafts undergo rejection because of mismatches in non-HLA molecules that function as minor antigens. This is also true for allogeneic induced pluripotent stem cells (iPSCs).

Hypoimmunogenic pluripotent (HIP) cells and cell products have gene knockouts or transgenes to protect them from the cellular components of the immune system that include T cells, NK cells, and macrophages. They may also be ABO blood group type O and Rh negative (HIPO-).

Transplanted cells may be killed by mechanisms involving antibodies are antibody-dependent cellular cytotoxicity (ADCC) by NK cells, macrophages, B-cells or granulocytes and complement-dependent cytotoxicity (CDC) via activation of the complement cascade. All of those killing mechanisms utilize antibodies that bind to a target cell and activate the effector immune cells or complement. Transplanted cells that comprise enhanced CD16, CD32, or CD64 expression may evade ADCC or CDC.

Polymorphonuclear cells (PMNs) are a category of white blood cells characterized by the presence of granules in their cytoplasm and lobed nuclei that are usually in three segments. This distinguishes them from the mononuclear immune cell fraction that contains lymphocytes, macrophages, and NK cells. Neutrophils are the most abundant of the PMNs, while eosinophils, basophils, and mast cells are less frequent PMNs that are found in the bloodstream. PMNs are the most abundant type of phagocytes, constituting 60% to 65% of the total circulating white blood cells. Once PMNs have received activation signals, it takes them about thirty minutes to leave the blood and reach the site of infection or tissue damage.

PMNs do not return to the blood. They exert their effector responses and die. PMNs have three mechanisms to attack, contain, and kill evading micro-organisms: 1) Phagocytosis. PMNs are professional phagocytes and rapidly engulf invaders coated with antibodies and complement, as well as damaged cells or cellular debris. 2) PMNs secrete cytotoxic granules. Containing reactive oxygen species, cationic proteins and defensins, lysozyme, myeloperoxidase, proteolytic enzymes and cathepsin G. 3) Formation of neutrophil extracellular traps (NETs). NETs comprise of a web of chromatin and serine proteases that trap and kill microbes extracellularly.

PMNs are often the first line of defense. They quickly migrate to sites of injury and infection and, because their defense mechanisms are very unspecific, may cause significant collateral damage to healthy cells in the vicinity. They get attracted and activated by cytokines, chemokines, pathogen-associated molecular patterns (PAMPs) and danger-associated molecular patterns (DAMPs). The latter activate pattern recognition receptors (PRRs) including toll-like receptors (TLRs). PMNs overall have multiple activating or inhibitory receptors.

Thus, transplanted cell death not only reduces the efficacy of the cell product but also triggers or amplifies innate defense mechanisms involving PMNs. PMN responses localized to the time and location of transplantation that contain and scavenge debris can also kill living cells in the vicinity as collateral damage. This further reduces cell survival and intensifies the PMN response. Strategies to prevent relevant PMN activation will facilitate cell transplantation and improve the potency of cell products. This invention outlines several points of action to control PMN activation that, separately or in combination, will improve cell transplantation.

## IV. SUMMARY OF THE INVENTION

The invention shows, for the first time, that activated PMNs kill cells derived from induced pluripotent stem cells *in vitro*. This includes unmodified and gene-edited hypoimmunogenic cells (HIP and HIPO- cells). This PMN barrier impedes the survival of transplanted cell products, including hypoimmunogenic cells, that otherwise evade peripheral blood mononuclear cell (PBMC) activation. The invention provides, for the first time, compositions and methods for evading PMN killing.

Thus, the invention provides a method of inhibiting polymorphonuclear (PMN) cells from killing a cell transplanted into a subject, comprising administering a PMN-inhibitory agent into the subject. In some aspects, the PMN inhibitory agent is an inflammatory inhibitor, a danger-associated molecular pattern (DAMP) inhibitor, a DAMP pathway inhibitor, a secretory enzyme release inhibitor, a reactive oxygen pathway inhibitor, a microtubule polymerization inhibitor, or a combination thereof.

In some aspects of the invention, the PMN-inhibitory agent is administered simultaneously with the transplanted cells or prior to the cell transplantation.

In some aspects, the PMN-inhitibory agent inhibits an interleukin 2 (IL-2), IL-2 receptor (IL-2R), toll-like receptor 4 (TLR4), TLR7, TLR8, TLR9, a toll-like receptor pathway, a neutrophil elastase (NE), a NADPH oxidase, microtubule polymerization, or wherein the PMN-inhitibory agent stimulates the cluster of differentiation 200 receptor (CD200R), PIRL$\alpha$, or SIRL-1.

In other aspects, the PMN-inhibitory agent is selected from the group consisting of cyclosporin A (CsA), dexamethasone, an anti-SIRL-1 antibody, a CD200 chimera, a CD200 expressed on cells, a CD99 chimera, a CD99 expressed on cells, BAY 85-8501, alpha-1 antitrypsin (A1AT), TAK-242, apocynin, idelalisib, CpG-52364, colchicine, and the anti-TLR4 antibody NI-0101.

In a preferred aspect, the method comprises the combination of an anti-SIRL-1 antibody and BAY 85-8501 or the combination of CsA and BAY 85-8501. In other preferred aspects, the method further comprises administering Colcicine, Idelalisib, or oligonucleotide CpG-52364. In some aspects, the combination of drugs work synergistically.

In some aspects of the invention, the PMN-inhibitory agent is expressed on the surface of the transplanted cells. In preferred aspects, the PMN-inhibitory agent is CD200 or CD99.

In some aspects of the invention, the transplanted cell is derived from a hypoimmune pluripotent (HIP) cell, a HIP cell that is AB blood group O and Rhesus Factor negative (HIPO-), an induced pluripotent stem cell (iPSC), an embryonic stem cell (ESC), or a derivative thereof. In other aspects of the invention, the transplanted cell is selected from the group consisting of a chimeric antigen receptor (CAR) cell, an endothelial cell, a dopaminergic neuron, a pancreatic islet cell, a cardiomyocyte, and a retinal pigment endothelium cell.

In some aspects of the invention, the transplanted cell is from a species selected from the group consisting of a human, monkey, cow, pig, chicken, turkey, horse, sheep, goat, donkey, mule, duck, goose, buffalo, camel, yak, llama, alpaca, mouse, rat, dog, cat, hamster, and guinea pig. In a preferred aspect, the transplanted cell is human.

The invention provides a pharmaceutical composition, comprising a cell derived from a pluripotent cell or a derivative thereof, a PMN-inhibitory agent, and a pharmaceutical excipient. In some aspects, the PMN inhibitory agent is an inflammatory inhibitor, a danger-associated molecular pattern (DAMP) inhibitor, or a combination thereof.

In some aspects of the invention, the PMN-inhibitory agent and the cell are delivered to a subject using separate delivery methods.

In some aspects of the invention, the PMN-inhitibory agent inhibits an interleukin 2 (IL-2) receptor (IL-2R), toll-like receptor 4 (TLR4), TLR7, TLR8, TLR9 or any of its downstream targets, neutrophil elastase (NE) receptor, NADPH-oxidase for superoxide production, or engages cluster of differentiation 200 receptor (CD200R), PIRL$\alpha$, or SIRL-1.

In some aspects of the invention, the PMN-inhibitory agent is selected from the group consisting of cyclosporin A (CsA), dexamethasone, an anti-SIRL-1 antibody, a CD200 chimera, a CD99 chimera, BAY 85-8501, alpha-1 antitrypsin (A1AT), TAK-242, apocynin, idelalisib, CpG-52364, colchicine, or the anti-TLR4 antibody NI-0101. In preferred aspects, the PMN-inhibitory agent comprises the combination of an anti-SIRL-1 antibody and BAY 85-8501 or the combination of CsA and BAY 85-8501. In other preferred aspects, the pharmaceutical composition further comprises Colcicine, Idelalisib, or oligonucleotide CpG-52364.

In some aspects of the invention, the PMN-inhibitory agent is expressed on the surface of the cell. In preferred aspects, the PMN-inhibitory agent is CD200 or CD99.

In some aspects of the invention, the pharmaceutical composition comprises a cell that is derived from a hypoimmune pluripotent (HIP) cell, a HIP cell that is AB blood group O, a Rhesus Factor negative (HIPO-), an induced pluripotent stem cell (iPSC), or an embryonic stem cell (ESC). In other aspects, the cell is selected from the group consisting of a chimeric antigen receptor (CAR) cell, an endothelial cell, a dopaminergic neuron, a pancreatic islet cell, a cardiomyocyte, and a retinal pigment endothelium cell.

In some aspects of the invention, the pharmaceutical composition comprises a transplanted cell that is from a species selected from the group consisting of a human, monkey, cow, pig, chicken, turkey, horse, sheep, goat, donkey, mule, duck, goose, buffalo, camel, yak, llama, alpaca, mouse, rat, dog, cat, hamster, and guinea pig. In a preferred aspect, the transplanted cell is human.

The invention provides a medicament for treating a disease, comprising a cell derived from a pluripotent cell or a derivative thereof, a PMN-inhibitory agent, and a pharmaceutical excipient. In some aspects, the derivative cell is selected from the group consisting of a chimeric antigen receptor (CAR) cell, an endothelial cell, a dopaminergic neuron, a pancreatic islet cell, a cardiomyocyte, and a retinal pigment endothelium cell. In other aspects, the disease is selected from the group consisting of Type I Diabetes, a cardiac disease, a neurological disease, a cancer, an ocular disease, and a vascular disease.

The invention provides a human hypo-immunogenic pluripotent (HIP) cell or a derivative thereof comprising: an overexpressed alpha-1 antitrypsin (A1AT) protein relative to a parent HIP cell; an endogenous Major Histocompatibility Antigen Class I (HLA-I) function that is reduced when compared to a parent pluripotent cell; an endogenous Major Histocompatibility Antigen Class II (HLA-II) function that is reduced when compared to the parent pluripotent cell; an increased CD47 function that reduces susceptibility to NK cell killing; an ABO blood group type O (O); and a Rhesus Factor (Rh) blood type negative (-); wherein the human hypo-immunogenic pluripotent O- (HIPO-) cell is less susceptible to rejection when transplanted into a subject when compared with an otherwise similar hypo-immunogenic pluripotent (HIP) cell that is an ABO blood group or Rh factor mismatch to the subject.

In some aspects of the invention, the A1AT protein comprises at least a 90% sequence identity to SEQ ID NO: 17. In other aspects, the A1AT protein comprises the the sequence of SEQ ID NO:17.

In some aspects of the invention, he HIPO- cell further comprises an elevated level of CD16, CD32, or CD64 protein expression when compared to a parent HIPO- cell, wherein the elevated protein expression causes the modified pluripotent cell to be less susceptible to antibody dependent cellular cytoxicity (ADCC) or complement-dependent cytotoxicity (CDC).

The invention provides a differentiated cell derived from the HIPO- cells overexpressing A1AT, wherein the differentiated cell is an endothelial cell or a fibroblast cell. In other aspects, the differentiated cell comprises an additional PMN-inhibitory agent and a pharmaceutical excipient. In preferred aspects, the additional PMN inhibitory agent is an inflammatory inhibitor, a danger-associated molecular pattern (DAMP) inhibitor, or a combination thereof. In other the additional PMN-inhibitory agent and the cell are delivered to a subject using separate delivery methods.

In other aspects, the PMN-inhitibory agent inhibits an interleukin 2 (IL-2) receptor (IL-2R), toll-like receptor 4 (TLR4), TLR7, TLR8, TLR9 or any of its downstream targets, neutrophil elastase (NE), NADPH-oxidase for superoxide production, or engages cluster of differentiation 200 receptor (CD200R), PIRL$\alpha$, or SIRL-1.

In other aspects of the invention, the PMN-inhibitory agent is selected from the group consisting of cyclosporin A (CsA), dexamethasone, an anti-SIRL-1 antibody, a CD200 chimera, a CD99 chimera, BAY 85-8501, TAK-242, apocynin, idelalisib, CpG-52364, colchicine, or the anti-TLR4 antibody NI-0101.

The invention provides a method of treating an alpha-1 antitrypsin deficiency (A1AD) in a subject comprising administering the pharmaceutical composition of the invention.

## V. **BRIEF DESCRIPTION OF THE DRAWINGS**

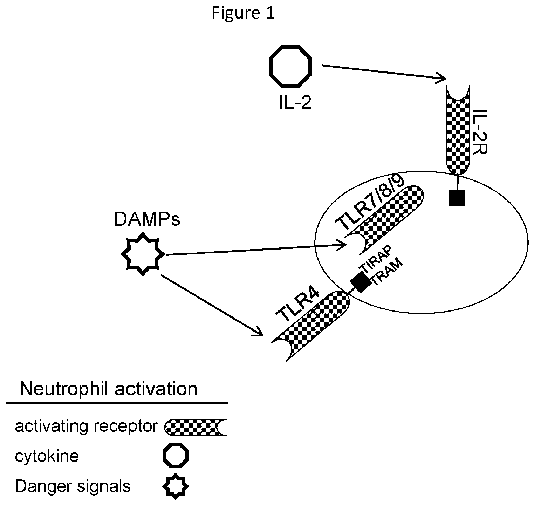

**Figure 1:** Schematic drawing of PMN activation by IL-2 or Danger Associated Molecular Patterns (DAMPs).

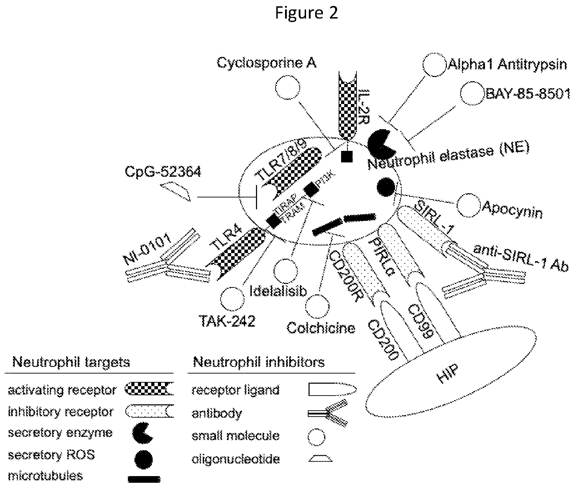

**Figure 2:** Summary of the PMN inhibitory strategies. PMN target categories are summarized on the left column of the legend. Different inhibitor classes are summarized on the right column of the legend. The name of exemplary inhibitors and their corresponding receptors or targets are specified in the figure.

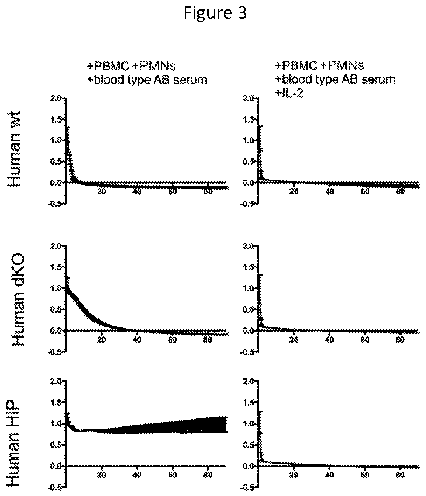

**Figure 3:** The XCelligence *in vitro* impedance system was used as a highly sensitive platform to obtain real-time kinetics of target cell killing. Wild-type (wt) and engineered iPSCs double knock-out (dKO: HLA class I and II) and hypoimmune pluripotent (HIP) cells were differentiated into endothelial cells for this assay. All components of the human immune system found in blood were included into an effector mix combining serum (contains antibodies, complement, and soluble factors like cytokines and growth factors), peripheral blood mononuclear cells (PBMCs, contains all lymphocytes, monocytes, and NK cells) and PMNs. Notably, the serum was taken from blood type AB donors and thus did not contain blood type antibodies. Some experimental groups were treated with IL-2 which is a central cytokine involved in allograft rejection. The assays were run for a duration of 90h. This is the maximum time period iECs can be cultured in this system before the monolayer breaks down spontaneously. XCelligence assays showed that viable allogeneic human wt iECs and dKO iECs were rejected *in vitro* in the absence of the pro-inflammatory cytokine IL-2. HIP cells were rejected only in the presence of IL-2. This shows that HIP iECs still undergo immune killing in an IL-2 environment.

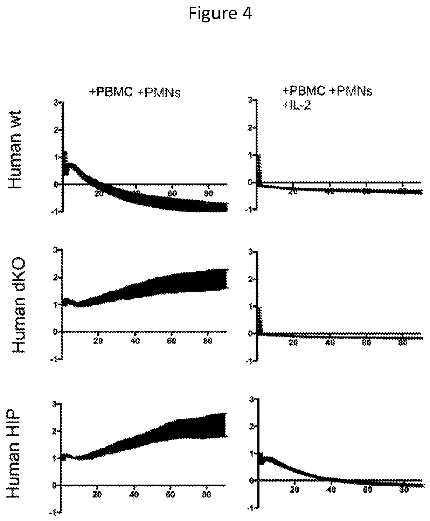

**Figure 4:** XCelligence assays run with allogeneic human PBMC and PMN effectors showed that HIP iECs were rejected in the presence of the proinflammatory cytokine IL-2 even in the absence of serum.

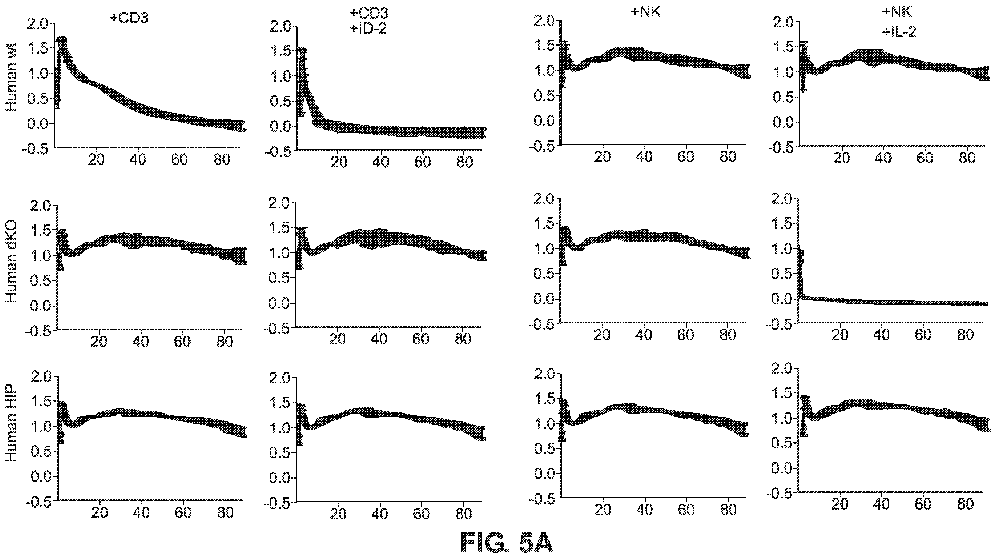

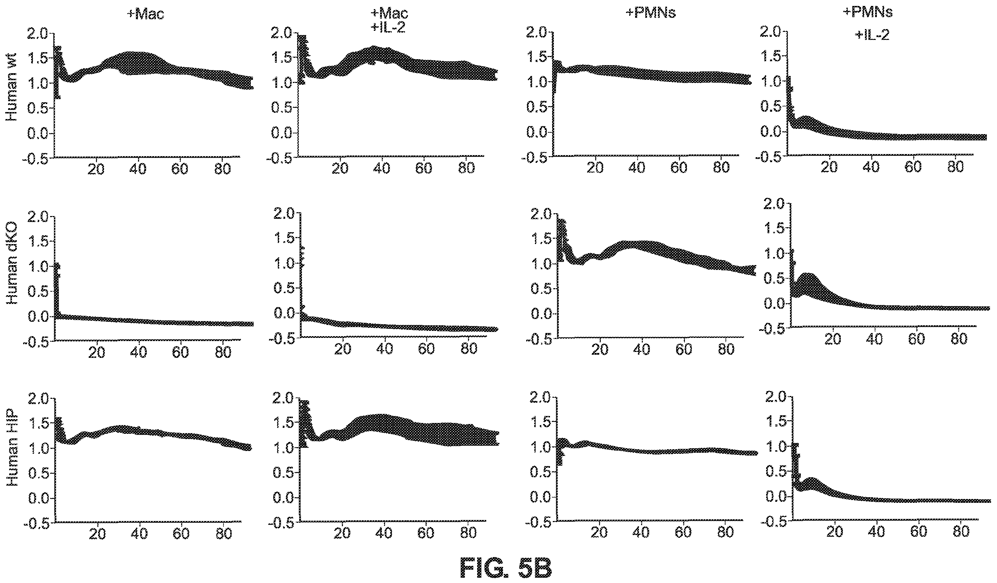

**Figure 5A:** XCelligence assays run with highly selected effector cell populations showed that neither CD3+ T cells nor natural killer cells could kill HIP iECs, even in the presence of the inflammatory IL-2 cytokine. Wild-type iECs were killed by allogeneic CD3+ cells and dKO iECs were killed by IL-2 activated NK cells. **Figure 5B:** HIP iECs were only killed by IL-2 activated PMNs but were resistant to macrophages (Mac) and quiescent PMNs.

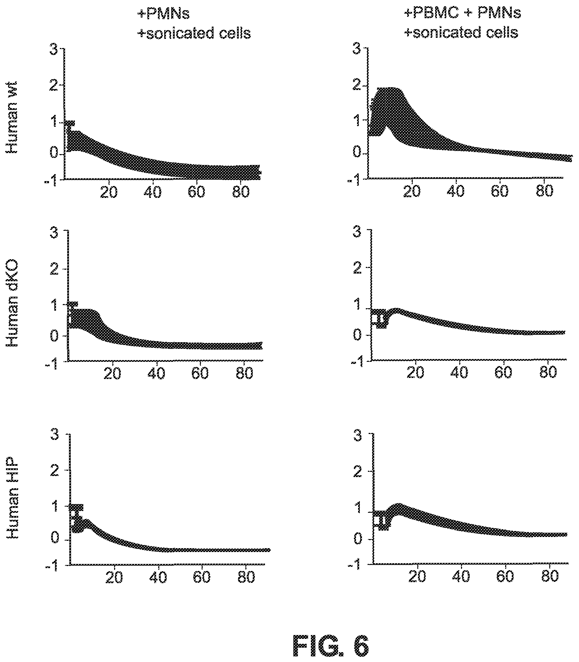

**Figure 6:** In this XCelligence assay, sonicated target cells were added to the wells at the time the immune cells were added to simulate cell necrosis with DAMP leakage. They showed that necrotic cells activated PMNs that in turn killed healthy target cells as collateral damage. When PBMCs were mixed with PMNs, the killing kinetics were slower. This was attributed to the 50% reduced PMN content in the mixed immune cell population.

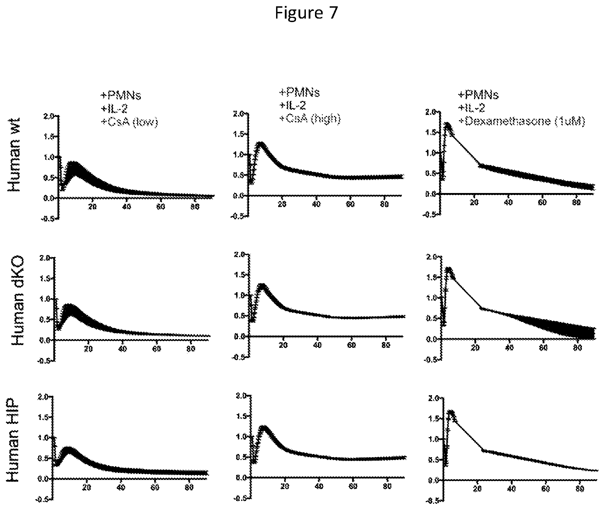

**Figure 7:** Cyclosporine and dexamethasone were tested for their efficacy to dampen a PMN killing response after IL-2 stimulation. XCelligence assays were performed with human wt, dKO, and HIP iECs against IL-2 stimulated human PMNs. Cyclosporine was used in two different concentrations. Cyclosporine and dexamethasone showed some minor efficacy to reduce PMN killing.

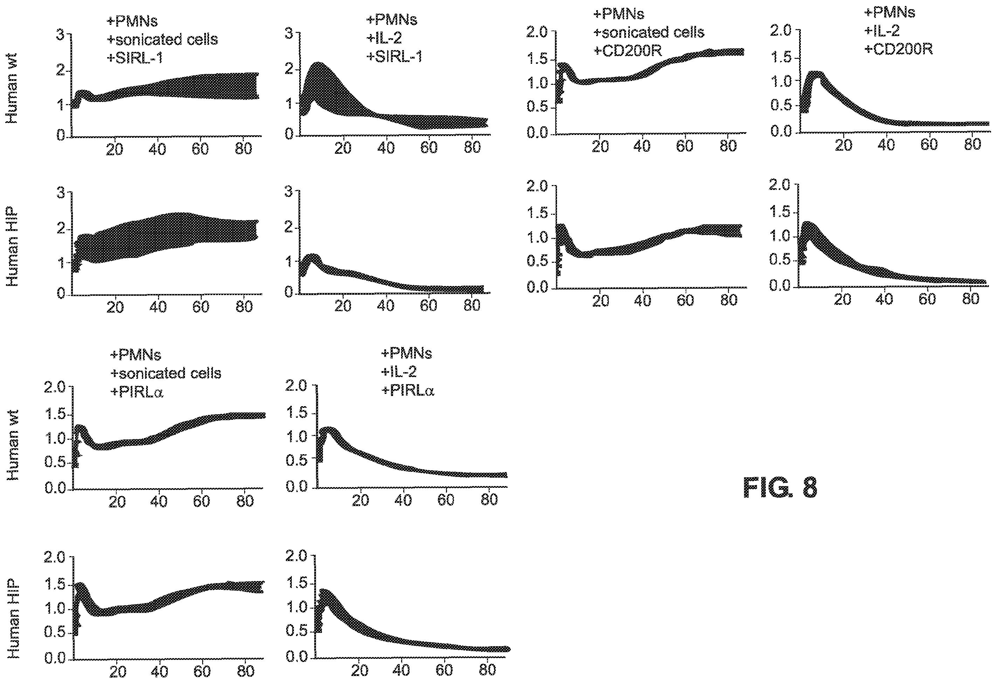

**Figure 8:** The human PMNs were activated via DAMPs or IL-2 and the killing of human wt and HIP_1.0 iECs was assessed by XCelligence assays. CD200R, PIRL$\alpha$, and SIRL-1 were engaged by recombinant CD200, CD99, or an inhibitory anti-SIRL-1 antibody, respectively. Engagement of those inhibitory receptors prevented PMN killing in the presence of necrotic cells. PMN killing was delayed but not prevented, however, if PMNs were stimulated with IL-2.

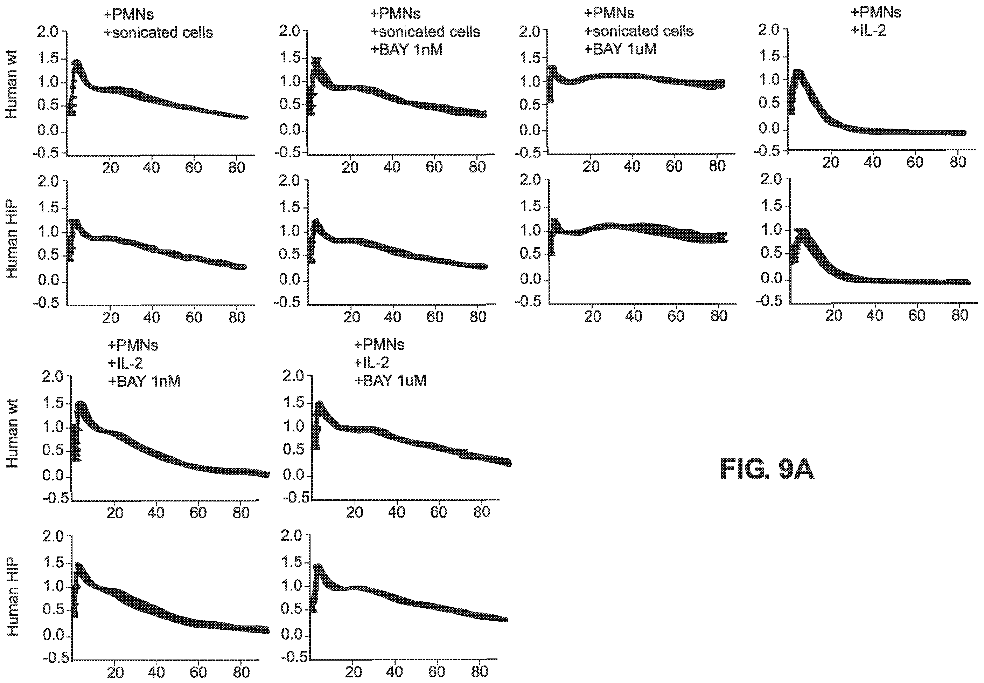

**Figure 9A:** Xcelligence assays were run in the presence of the neutrophil elastase (NE) inhibitor BAY 85-8501. $1\mu$M BAY 85-8501 fully prevented human wt or HIP target cell killing when PMNs were activated via DAMPs from sonicated cells. The same concentration of BAY 85-8501 further delayed the target cell killing in the presence of IL-2. BAY 85-8501 at 1nM was less effective.

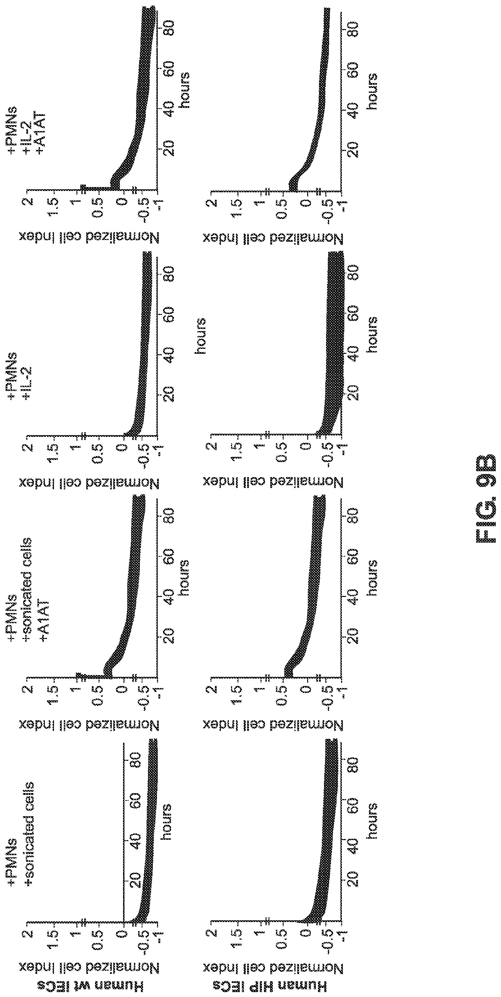

**Figure 9B:** Xcelligence assays were run in the presence of the NE inhibitor alpha-1 antitrypsin (A1AT). Recombinant A1AT showed some minor activity against PMN killing of human wt and HIP iECs.

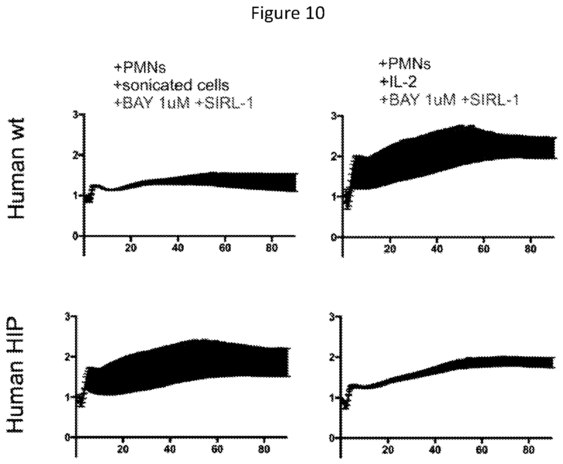

**Figure 10:** The anti-SIRL-1 antibody in combination with BAY 85-8501 was a highly potent PMN inhibitor regimen in XCelligence assays with human PMNs activated via DAMPs or IL-2.

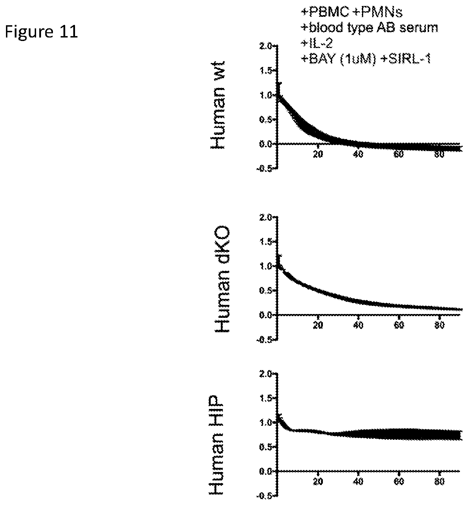

**Figure 11:** BAY 85-8501 with the anti-SIRL mAb was highly efficacious in an XCelligence assay that combined all human immune system components PBMCs, PMNs, and serum in a pro-inflammatory IL-2 environment

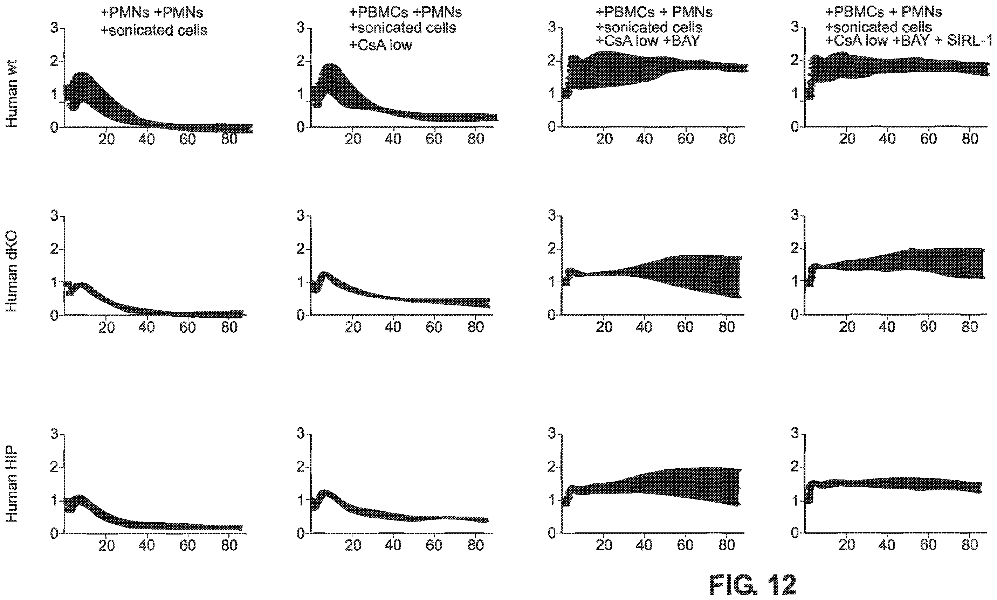

**Figure 12:** Cyclosprorin A (CsA) combined with BAY 85-8501 was confirmed in the presence of PBMCs and PMNs using the XCelligence assay. Low-dose CsA was combined with BAY 85-8501, with or without the anti-SIRL-1 mAb, to prevent killing of all target cells using PBMCs and PMNs as effector cells. Necrotic cells were used as stimulators. The combinations of low-dose CsA, BAY 85-8501 and anti-SIRL-1 mAb, but also low-dose CsA and BAY 85-8501 were highly effective in preventing killing of all target cells.

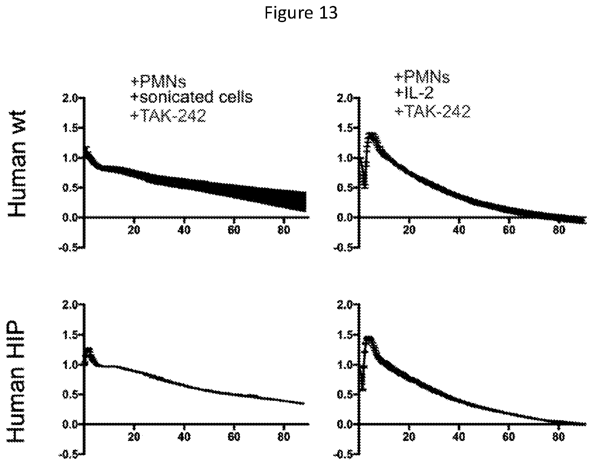

**Figure 13:** PMN activation pathways were inhibited by TAK-242, a small-molecule inhibitor of TLR-4-mediated signaling. Wt and HIP iECs were used on the XCelligence platform using PMNs as effector cells and DAMPs or IL-2 as PMN stimulators. Inhibition of TLR-4 signaling with TAK-242 diminished PMN killing. The efficacy was stronger with the somewhat weaker stimulatory signal via DAMPs. With IL-2, PMN killing was still markedly reduced.

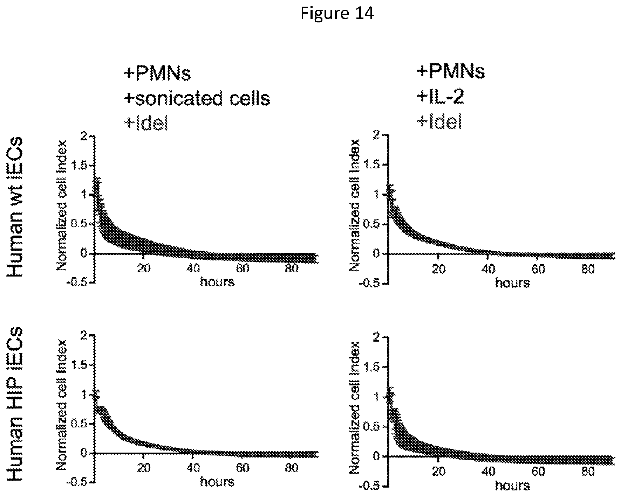

**Figure 14:** PMN inhibition via TLR-4 signaling was assessed using Idelalisib, a downstream inhibitor. Inhibition of TLR-4 signaling with Idelalisib mildly diminished PMN killing.

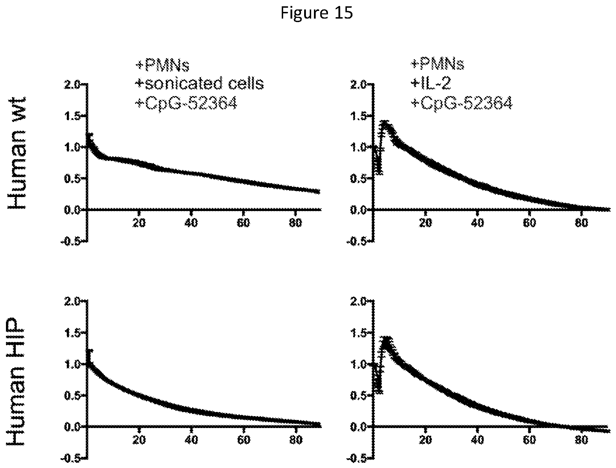

**Figure 15:** Inhibition of TLR7/8/9 signaling was tested using the oligonucleotide CpG-52364. Wt and HIP iECs were used in XCelligence PMN assays using necrotic cells or IL-2 as stimulators. Inhibition of TLR-7/8/9 signaling with CpG-52364 also reduced PMN killing. CpG-52364 was more effective when sonicated cells were used as stimlators compared to IL-2.

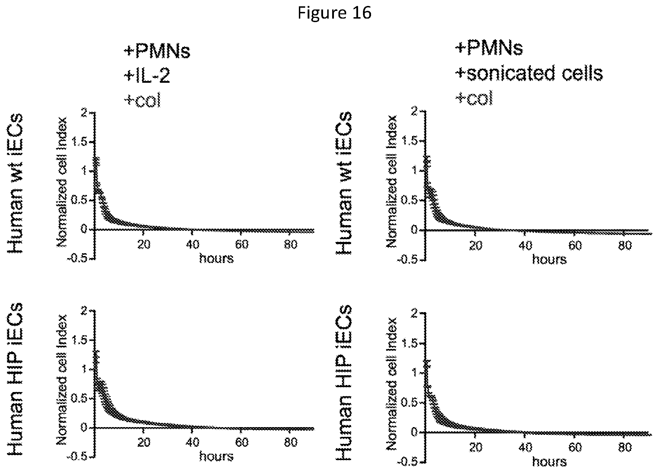

**Figure 16:** Colchicine (col) was used here as another strategy to inhibit PMN killing as a monotherapy. In Xcelligence assays with DAMP or IL-2 activated PMNs, the monotherapy of colchicine showed only marginal efficacy.

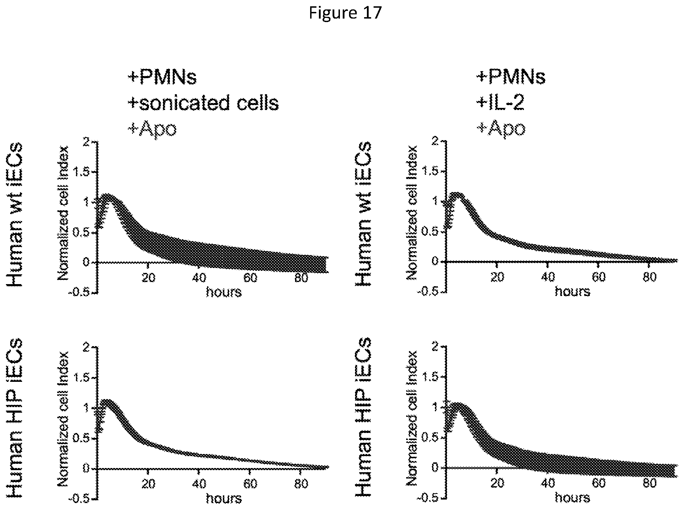

**Figure 17:** Apocynin (Apo) diminished DAMP- or IL-2 activated PMN killling. Apocynin reduced PMN killing for wt and HIP iEC target cells

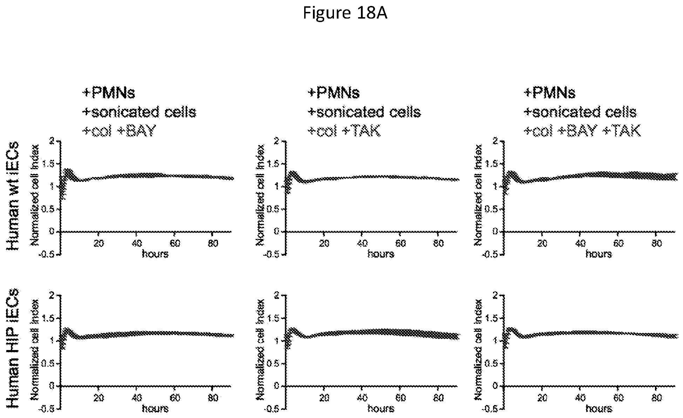

**Figure 18A:** Combination regimens were used to increase the overall efficacy of PMN inhibition. PMN XCelligence assays were run with combinations of colchicine (col), BAY 85-8501, or TAK-242. DAMPs were used to activate PMNs. All combinations were very effective to completely inhibit PMN killing.

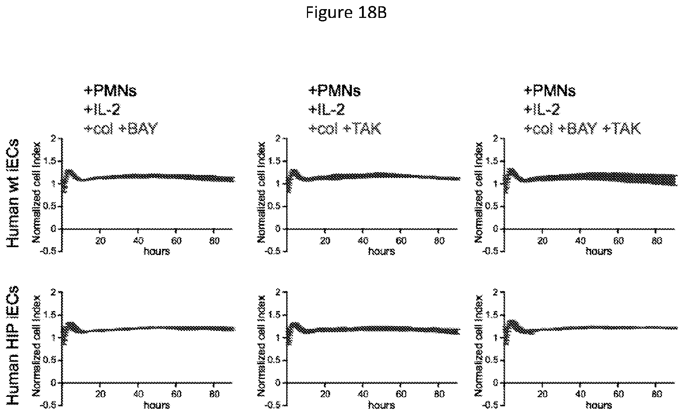

**Figure 18B:** Combination regimens were used to increase the overall efficacy of PMN inhibition. PMN XCelligence assays were run with combinations of colchicine (col), BAY 85-8501, or TAK-242. IL-2 was used to activate PMNs. All combinations were very effective to completely inhibit PMN killing.

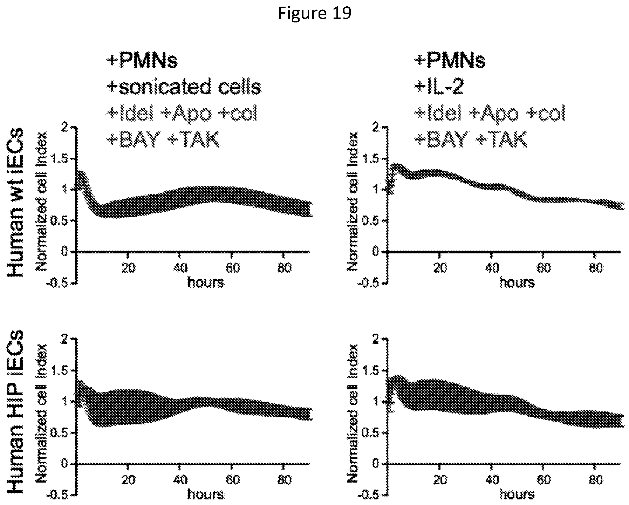

**Figure 19:** PMN XCelligence assays were run with a combination of Idelalisib (Idel), Apocynin (Apo), colchicine (col), BAY 85-8501, and TAK-242. DAMPs or IL-2 was used to activate PMNs. This combination was very effective to completely inhibit PMN killing.

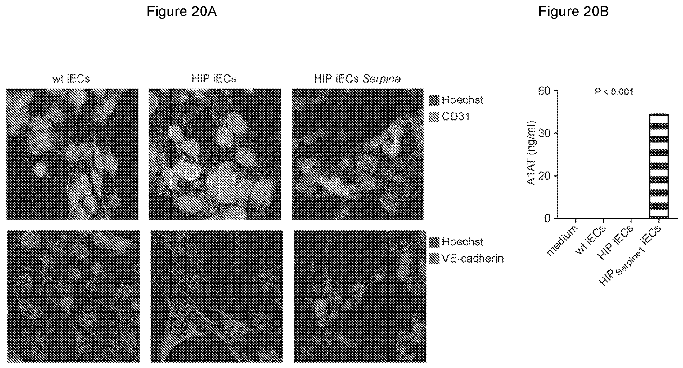

**Figure 20A:** A1AT-releasing HIP$_{Serpine1}$ iECs. HIP iECs engineered to make A1AT (HIP$_{Serpine1}$ iECs) were grown in culture and shared typical endothelial cell features with their parental wt iECs and HIP iECs (representative pictures of two independent experiments).

**Figure 20B:** A1AT-releasing HIP$_{Serpine1}$ iECs. A total of $1.5 \times 10^5$ wt, HIP, and HIP$_{Serpine1}$ iECs were cultured for 24 h in endothelial cell medium. The A1AT concentration was then assessed by Elisa (mean $\pm$ s.d., three independent experiments, analysis of variance (ANOVA)).

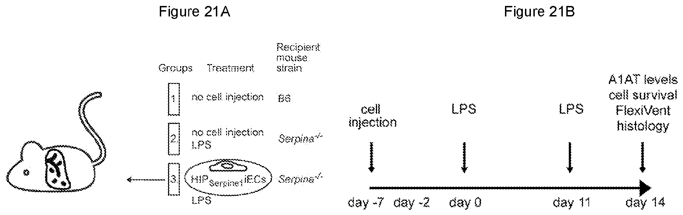

**Figure 21A:** Study design for A1AT-releasing HIPSerpine1 iEC therapy. *Serpina*$^{-/-}$ mice were challenged with LPS via their airways to trigger emphysematous lung disease. Healthy B6 animals served as controls. The study protocol included morphologic as well as functional lung assessment after 28 days.

**Figure 21B:** Study design for A1AT-releasing HIPSerpine1 iEC therapy. Group 3 animals were treated with HIP$_{Serpine1}$ iECs 7 days before the LPS challenge

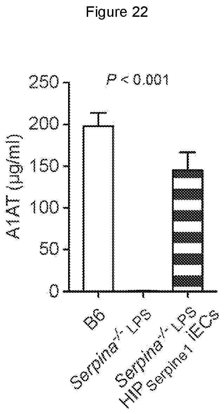

**Figure 22:** *Serum A1AT levels.* On day 21 after HIP$_{Serpine1}$ iEC transplantation (14 days after the first LPS challenge), serum A1AT levels were quantified in all groups (mean ± s.d., 6 animals in B6, 5 animals in *Serpina$^{-/-}$* LPS, 6 animals in *Serpina$^{-/-}$* LPS HIP$_{Serpine1}$ iEC group, analysis of variance (ANOVA)).

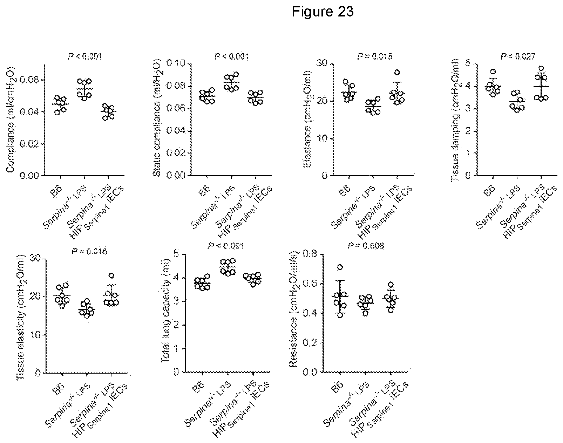

**Figure 23:** Assessment of pulmonary mechanics. FlexiVent lung physiology assessments were done after 28 days (scatter dot plots, mean ± s.d., 6 animals per group, analysis of variance (ANOVA)). Untreated *Serpina$^{-/-}$* mice developed ventilation patterns consistent with developing emphysema. HIP$_{Serpine1}$ iEC therapy was effective in preventing the development of respiratory patterns typical of emphysematous lung disease in *Serpina$^{-/-}$* mice.

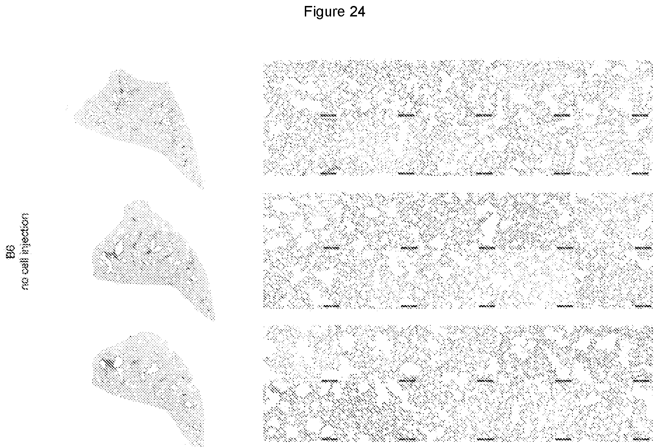

**Figure 24:** Assessment of airspace characteristics in healthy mouse lungs. Lungs of healthy B6 mice were sectioned, stained, and underwent stereological assessment (representative pictures of 6 animals).

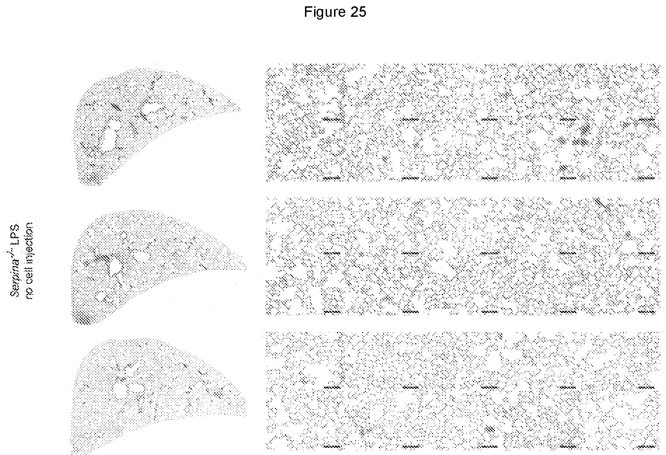

**Figure 25:** Assessment of airspace characteristics in Serpina$^{-/-}$ mice. Serpina$^{-/-}$ mice were exposed to a mild LPS airways challenge and developed features of emphysema (representative pictures of six animals).

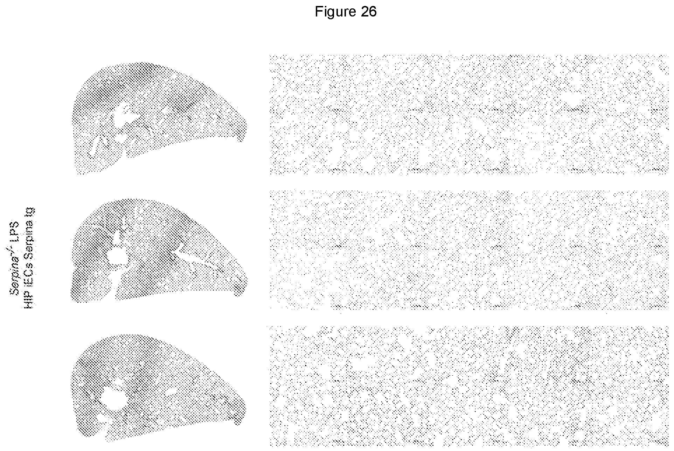

**Figure 26:** Assessment of airspace characteristics in treated Serpina$^{-/-}$ mice. Serpina$^{-/-}$ mice received $1.5 \times 10^8$ HIP$_{Serpine1}$ iECs 7 days before the first LPS challenge (representative pictures of 6 animals).

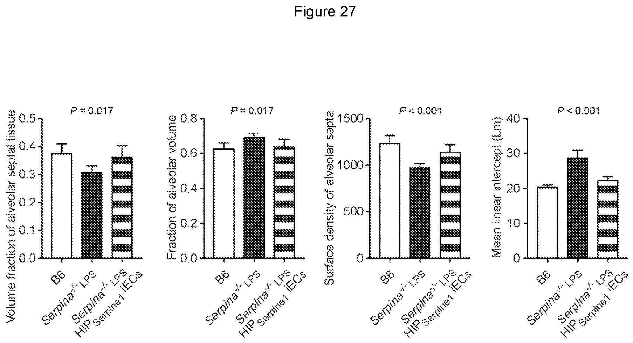

**Figure 27:** Stereological lung assessment. Histology slides were scanned and assessed in a standardized way. Stereological lung assessment (mean ± s.d., six animals per group, analysis of variance (ANOVA)). Morphometric criteria for emphysema in untreated *Serpina$^{-/-}$* mice including distal airspace enlargement, loss of alveoli and alveolar surface area, and increase in mean linear intercept (chord) length, a parameter to describe the mean free distance in the air spaces were found. HIP$_{Serpine1}$ iEC therapy was successful in preventing all structural damages to the lung tissue.

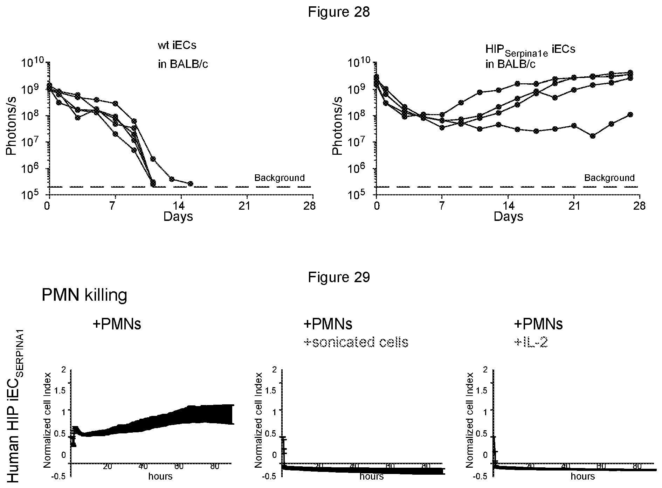

**Figure 28:** Allogeneic BALB/c recipients were used to separately assess graft survival. Wild type B6 iECs did not survive allogeneic transplantation into BALB/c mice beyond 14 days. In contrast HIP$_{Serpine1}$ iEC from B6 mice survived allogenic transplantation in BALB/c mice for at least 28 days.

**Figure 29:** Human HIP iEC$_{SERPINA1}$ target cells on the XCelligence platform were not killed by human PMNs. Both sonicated target cells and IL-2, however, led to PMN activation and target HIP iEC$_{SERPINA1}$ killing.

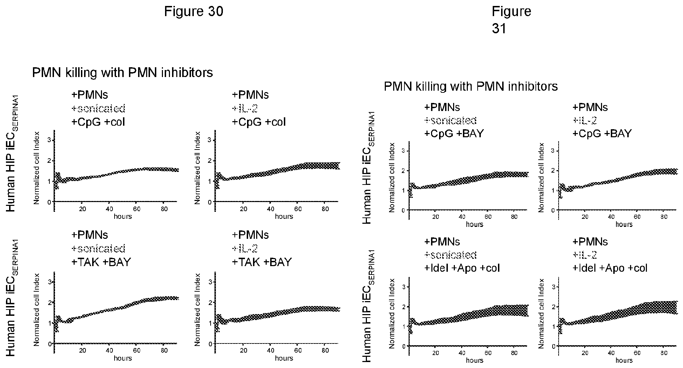

**Figure 30:** Human HIP iEC$_{SERPINA1}$ target cells on the XCelligence platform were challenged with PMNs activated via sonicated target cells (left column) or IL-2 (right column). The HIP iEC$_{SERPINA1}$ target cells were not killed when two PMN inhibitory regimens were used: CpG-52364 + colchicine (upper row) and TAK-242 + BAY 85-8501.

**Figure 31:** Human HIP iEC$_{SERPINA1}$ target cells on the XCelligence platform were challenged with PMNs activated via sonicated target cells (left column) or IL-2 (right column). The HIP iEC$_{SERPINA1}$ target cells were also not killed when two other PMN inhibitory regimens were used: CpG-52364 + BAY 85-8501 (upper row) and Idelalisib + Apocynin + colchicine.

## VI. DETAILED DESCRIPTION OF THE INVENTION

A. Introduction

The invention identifies PMNs as an additional immune barrier for the survival of cell transplantation. Non-specific DAMPs released by necrotic cells can induce PMN activation and lead to the killing of healthy bystander cells as collateral damage in an attempt to contain and clear infection or injury. This killing is independent of hypoimmune (e.g. HIP) cell gene editing and relates to the cell transplant period prior to when the cells have integrated and engrafted into the host tissue. Non-stimulated PMNs show no tendency to kill healthy allogeneic target cells.

The invention provides, for the first time, regenerative cell therapies having a reduced sensitivity to the cell transplant recipient's innate immune system that kills the cells during the window of time shortly after transplantation. In some embodiments, the invention blocks PMN cell killing induced by cytokines, chemokines, danger-associated molecular patterns (DAMPs, Figure 1), or pathogen-associated molecular patterns (PAMPs).

A summary of the PMN inhibitory strategies is shown at Figure 2. In some embodiments, PMNs are inhibited by targeting the CD200 Receptor (CD200R), PIRLa, SIRL-1, Neutrophil Elastase (NE), IL-2R, TLR4, TLR7, TLR8, TLR9, microtubule polymerization, or superoxide release. In other embodiments, transplanted cells such as hyipoimmune pluripotent cells (HIP), or derivatives thereof, that express increased CD200 or CD99.

Neutrophil Elastase is a serine proteinase in the same family as chymotrypsin and has broad substrate specificity. Secreted by neutrophils and macrophages during inflammation, it destroys bacteria and host tissue. It also localizes to Neutrophil extracellular traps (NETs) via its high affinity for DNA, an unusual property for serine proteases.

The invention provides effective regimens to dampen or even fully prevent PMN killing of human target cells. PMN activation and response is complex and multi-layered. While single drugs were effective, combinations of druges were more likely to completely inhibit PMN killing. In some embodiments, the drug combinations are synergistic. Exemplary embodiments include:

- TAK-242 and CpG-52364 are very effective to dampen PMN activation, likely via parenteral application.

- Idelalisib and apocynin are available for oral application and is effective to dampen PMN activation.

- Colchicine is available for oral application, acts via a mechanism distinct of all other drugs, and ample human data are available.

- BAY 85-8501 is available for oral application and shows high selectivity and efficacy for the inhibition of PMN killing.

- NI-0101 is available for parenteral application and is effective to dampen PMN activation.

- Ciclosporine A is a calcineurin inhibitor like the more commonly used tacrolimus and both will be able to reduce IL-2, a very strong stimulator of PMN activation.

The inhibitory PMN strategies provided herein may only be necessary during the cell transplant and engraftment period when strong activating PMN signals are present and no further treatment is necessary for maintianiang hypoimmune cells or their derivatives. Where permanent PMN inhibition is desired, the invention provides modified cells with increased CD200 or CD99 surface expression or cells made to release Alpha1 Antitrypsin.

In some embodiments of the invention, **H**ypo**I**mmunogenic **P**luripotent ("HIP") cells, or derivatives thereof, are transplanted in combination with PMN cell-inhibitory strategy. HIP cells avoid host immune responses due to several genetic manipulations. The cells lack major immune antigens that trigger immune responses and are engineered to avoid phagocytosis and NK cell killing. In some embodiments, the HIP cells are made by eliminating the activity of both alleles of a B2M gene in an induced pluripotent stem cell (iPSC); eliminating the activity of both alleles of a CIITA gene in the iPSC; and increasing the expression of CD47 in the iPSC. HIP cells are described in detail in WO2018132783, incorporated by reference herein in its entirety.

In some embodiments of the invention, **H**ypo**I**mmunogenic **P**luripotent Blood group **O** Rh - ("HIPO-") cells, or derivatives thereof, are transplanted in combination with a PMN cell-inhibitory strategy. HIPO- cells avoid host immune responses due to several genetic or enzymatic manipulations. The cells lack major blood group and immune antigens that trigger immune responses and are engineered to avoid rejection, phagocytosis, or killing. This allows the derivation of "off-the-shelf" cell products for generating specific tissues and organs. The benefit of being able to use human allogeneic HIPO- cells and their derivatives in human patients provides significant benefits, including the ability to avoid long-term adjunct immunosuppressive therapy and drug use generally seen in allogeneic transplantations. They also provide significant cost savings as cell therapies can be used without requiring individual treatments for each patient.

HIPO-/CD16, HIPO-/CD32, or HIPO-/CD64 cells may serve as a universal cell source for the generation of universally-acceptable derivatives. HIPO- cells are described in detail in U.S. Prov. Appl. Nos. 62/846,399 and 62/855,499. Transplanted cells that comprise enhanced CD16, CD32, or CD64 expression to evade ADCC or CDC are described in 62/915,601. The foregoing are incorporated by reference herein in their entirety.

In some embodiments, the transplanted cells comprise enhanced CD16, CD32, or CD64 expression to evade antibody-dependent cellular cytotoxicity (ADCC) or complement-dependent cytotoxicity (CDC). Such cells are described in detail in U.S. Prov. Appl. No. 62/915,601, incorporated by reference herein in its entirety.

B. Definitions

The term "pluripotent cells" refers to cells that can self-renew and proliferate while remaining in an undifferentiated state and that can, under the proper conditions, be induced to differentiate into specialized cell types. The term "pluripotent cells," as used herein, encompass embryonic stem cells and other types of stem cells, including fetal, amnionic, or somatic stem cells. Exemplary human stem cell lines include the H9 human embryonic stem cell line. Additional exemplary stem cell lines include those made available through the National Institutes of Health Human Embryonic Stem Cell Registry and the Howard Hughes Medical Institute HUES collection (as described in Cowan, C. A. et. al, New England J. Med. 350:13. (2004), incorporated by reference herein in its entirety.)

"Pluripotent stem cells" as used herein have the potential to differentiate into any of the three germ layers: endoderm (*e.g.* the stomach linking, gastrointestinal tract, lungs, etc), mesoderm (*e.g.* muscle, bone, blood, urogenital tissue, etc) or ectoderm (*e.g.* epidermal tissues and nervous system tissues). The term "pluripotent stem cells," as used herein, also encompasses "induced pluripotent stem cells", or "iPSCs", a type of pluripotent stem cell derived from a non-pluripotent cell. Examples of parent cells include somatic cells that have been reprogrammed to induce a pluripotent, undifferentiated phenotype by various means. Such "iPS" or "iPSC" cells can be created by inducing the expression of certain regulatory genes or by the exogenous application of certain proteins. Methods for the induction of iPS cells are known in the art and are further described below. (See, e.g., Zhou et al., Stem Cells 27 (11): 2667-74 (2009); Huangfu et al., Nature Biotechnol. 26 (7): 795 (2008); Woltjen et al., Nature 458 (7239): 766-770 (2009); and Zhou et al., Cell Stem Cell 8:381-384 (2009); each of which is incorporated by reference herein in their entirety.) The generation of induced pluripotent stem cells (iPSCs) is outlined below. As used herein, "hiPSCs" are human induced pluripotent stem cells, and "miPSCs" are murine induced pluripotent stem cells.

"Pluripotent stem cell characteristics" refer to characteristics of a cell that distinguish pluripotent stem cells from other cells. The ability to give rise to progeny that can undergo differentiation, under the appropriate conditions, into cell types that collectively demonstrate characteristics associated with cell lineages from all of the three germinal layers (endoderm, mesoderm, and ectoderm) is a pluripotent stem cell characteristic. Expression or non-expression of certain combinations of molecular markers are also pluripotent stem cell characteristics. For example, human pluripotent stem cells express at least several, and in some embodiments, all of the markers from the following non-limiting list: SSEA-3, SSEA-4, TRA-1-60, TRA-1-81, TRA-2-49/6E, ALP, Sox2, E-cadherin, UTF-1, Oct4, Rex1, and Nanog. Cell morphologies associated with pluripotent stem cells are also pluripotent stem cell characteristics. As described herein, cells do not need to pass through pluripotency to be reprogrammed into endodermal progenitor cells and/or hepatocytes.

As used herein, "multipotent" or "multipotent cell" refers to a cell type that can give rise to a limited number of other particular cell types. For example, induced multipotent cells are capable of forming endodermal cells. Additionally, multipotent blood stem cells can differentiate itself into several types of blood cells, including lymphocytes, monocytes, neutrophils, etc.

As used herein, the term "oligopotent" refers to the ability of an adult stem cell to differentiate into only a few different cell types. For example, lymphoid or myeloid stem cells are capable of forming cells of either the lymphoid or myeloid lineages, respectively.

As used herein, the term "unipotent" means the ability of a cell to form a single cell type. For example, spermatogonial stem cells are only capable of forming sperm cells.

As used herein, the term "totipotent" means the ability of a cell to form an entire organism. For example, in mammals, only the zygote and the first cleavage stage blastomeres are totipotent.

As used herein, "non-pluripotent cells" refer to mammalian cells that are not pluripotent cells. Examples of such cells include differentiated cells as well as progenitor cells. Examples of differentiated cells include, but are not limited to, cells from a tissue selected from bone marrow, skin, skeletal muscle, fat tissue and peripheral blood. Exemplary cell types include, but are not limited to, fibroblasts, hepatocytes, myoblasts, neurons, osteoblasts, osteoclasts, and T-cells. The starting cells employed for generating the induced multipotent cells, the endodermal progenitor cells, and the hepatocytes can be non-pluripotent cells.

Differentiated cells include, but are not limited to, multipotent cells, oligopotent cells, unipotent cells, progenitor cells, and terminally differentiated cells. In particular embodiments, a less potent cell is considered "differentiated" in reference to a more potent cell.

A "somatic cell" is a cell forming the body of an organism. Somatic cells include cells making up organs, skin, blood, bones and connective tissue in an organism, but not germ cells.

Cells can be from, for example, human or non-human mammals. Exemplary non-human mammals include, but are not limited to, mice, rats, cats, dogs, rabbits, guinea pigs, hamsters, sheep, pigs, horses, bovines, and non-human primates. In some embodiments, a cell is from an adult human or non-human mammal. In some embodiments, a cell is from a neonatal human, an adult human, or non-human mammal.

As used herein, the terms "subject" or "patient" refers to any animal, such as a domesticated animal, a zoo animal, or a human. The "subject" or "patient" can be a mammal like a dog, cat, bird, livestock, or a human. Specific examples of "subjects" and "patients" include, but are not limited to, individuals (particularly human) with a disease or disorder related to the liver, heart, lung, kidney, pancreas, brain, neural tissue, blood, bone, bone marrow, and the like.

Mammalian cells can be from humans or non-human mammals. Exemplary non-human mammals include, but are not limited to, mice, rats, cats, dogs, rabbits, guinea pigs, hamsters, sheep, pigs, horses, bovines, and non-human primates (e.g., chimpanzees, macaques, and apes).

By "hypo-immunogenic pluripotent" cell or "HIP" cell herein is meant a pluripotent cell that retains its pluripotent characteristics and yet gives rise to a reduced immunological rejection response when transferred into an allogeneic host. In preferred embodimements, HIP cells do not give rise to an immune response. Thus, "hypo-immunogenic" refers to a significantly reduced or eliminated immune response when compared to the immune response of a parental (*i.e.* "wt") cell prior to immunoengineering as outlined herein. In many cases, the HIP cells are immunologically silent and yet retain pluripotent capabilities. Assays for HIP characteristics are outlined below.

By "HIP/CD16", "HIP/CD32", or "HIP/CD64" cell herein is meant a HIP cell that has enhanced CD16, CD32, or CD64 expression, respectively.

By "hypo-immunogenic pluripotent cell O-" "hypo-immunogenic pluripotent ORh-" cell or "HIPO-" cell herein is meant a HIP cell that is also ABO blood group O and Rhesus Factor Rh-. HIPO- cells may have been generated from O-cells, enzymatically modified to be O-, or genetically engineered to be O-.

By "HIPO-/CD16", "HIPO-/CD32", or "HIPO-/CD64" cell herein is meant a HIPO-cell that has enhanced CD16, CD32, or CD64 expression.

By "HLA" or "human leukocyte antigen" complex is a gene complex encoding the major histocompatibility complex (MHC) proteins in humans. These cell-surface proteins that make up the HLA complex are responsible for the regulation of the immune response to antigens. In humans, there are two MHCs, class I and class II, "HLA-I" and "HLA-II". HLA-I includes three proteins, HLA-A, HLA-B and HLA-C, which present peptides from the inside of the cell, and antigens presented by the HLA-I complex attract killer T-cells (also known as CD8+ T-cells or cytotoxic T cells). The HLA-I proteins are associated with β-2 microglobulin (B2M). HLA-II includes five proteins, HLA-DP, HLA-DM, HLA-DOB, HLA-DQ and HLA-DR, which present antigens from outside the cell to T lymphocytes. This stimulates CD4+ cells (also known as T-helper cells). It should be understood that the use of either "MHC" or "HLA" is not meant to be limiting, as it depends on whether the genes are from humans (HLA) or murine (MHC). Thus, as it relates to mammalian cells, these terms may be used interchangeably herein.

By "gene knock out" herein is meant a process that renders a particular gene inactive in the host cell in which it resides, resulting either in no protein of interest being produced or an inactive form. As will be appreciated by those in the art and further described below, this can be accomplished in a number of different ways, including removing nucleic acid sequences from a gene, or interrupting the sequence with other sequences, altering the reading frame, or altering the regulatory components of the nucleic acid. For example, all or part of a coding region of the gene of interest can be removed or replaced with "nonsense" sequences, all or part of a regulatory sequence such as a promoter can be removed or replaced, translation initiation sequences can be removed or replaced, etc.

By "gene knock in" herein is meant a process that adds a genetic function to a host cell. This causes increased levels of the encoded protein. As will be appreciated by those in the art, this can be accomplished in several ways, including adding one or more additional copies of the gene to the host cell or altering a regulatory component of the endogenous gene increasing expression of the protein is made. This may be accomplished by modifying the promoter, adding a different promoter, adding an enhancer, or modifying other gene expression sequences.

"β-2 microglobulin" or "β2M" or "B2M" protein refers to the human β2M protein that has the amino acid and nucleic acid sequences shown below; the human gene has accession number NC_000015.10:44711487-44718159.

"CD47 protein" protein refers to the human CD47 protein that has the amino acid and nucleic acid sequences shown below; the human gene has accession number NC_000016.10:10866208-10941562.

"CIITA protein" protein refers to the human CIITA protein that has the amino acid and nucleic acid sequences shown below; the human gene has accession number NC_000003.12:108043094-108094200.

By "wild type" in the context of a cell means a cell found in nature. However, in the context of a pluripotent stem cell, as used herein, it also means an iPSC that may contain nucleic acid changes resulting in pluripotency but did not undergo the gene editing procedures of the invention to achieve hypo-immunogenicity.

By "syngeneic" herein refers to the genetic similarity or identity of a host organism and a cellular transplant where there is immunological compatibility; *e.g.* no immune response is generated.

By "allogeneic" herein refers to the genetic dissimilarity of a host organism and a cellular transplant where an immune response is generated.

By "B2M-/-" herein is meant that a diploid cell has had the B2M gene inactivated in both chromosomes. As described herein, this can be done in a variety of ways.

By "CIITA -/-" herein is meant that a diploid cell has had the CIITA gene inactivated in both chromosomes. As described herein, this can be done in a variety of ways.

By "CD47 tg" (standing for "transgene") or "CD47+") herein is meant that the host cell expresses CD47, in some cases by having at least one additional copy of the CD47 gene.

An "Oct polypeptide" refers to any of the naturally-occurring members of Octamer family of transcription factors, or variants thereof that maintain transcription factor activity, similar (within at least 50%, 80%, or 90% activity) compared to the closest related naturally occurring family member, or polypeptides comprising at least the DNA-binding domain of the naturally occurring family member, and can further comprise a transcriptional activation domain. Exemplary Oct polypeptides include Oct-1, Oct-2, Oct-3/4, Oct-6, Oct-7, Oct-8, Oct-9, and Oct-11. Oct3/4 (referred to herein as "Oct4") contains the POU domain, a 150 amino acid sequence conserved among Pit-1, Oct-1, Oct-2, and uric-86. (See, Ryan, A. K. & Rosenfeld, M. G., Genes Dev. 11:1207-1225 (1997), incorporated herein by reference in its entirety.) In some embodiments, variants have at least 85%, 90%, or 95% amino acid sequence identity across their whole sequence compared to a naturally occurring Oct polypeptide family member such as to those listed above or such as listed in Genbank accession number NP-002692.2 (human Oct4) or NP-038661.1 (mouse Oct4). Oct polypeptides (e.g., Oct3/4 or Oct 4) can be from human, mouse, rat, bovine, porcine, or other animals. Generally, the same species of protein will be used with the species of cells being manipulated. The Oct polypeptide(s) can be a pluripotency factor that can help induce multipotency in non-pluripotent cells.

A "Klf polypeptide" refers to any of the naturally-occurring members of the family of Krüppel-like factors (Klfs), zinc-finger proteins that contain amino acid sequences similar to those of the Drosophila embryonic pattern regulator Krüppel, or variants of the naturally-occurring members that maintain transcription factor activity similar (within at least 50%, 80%, or 90% activity) compared to the closest related naturally occurring family member, or polypeptides comprising at least the DNA-binding domain of the naturally occurring family member, and can further comprise a transcriptional activation domain. (See, Dang, D. T., Pevsner, J. & Yang, V. W., Cell Biol. 32:1103-1121 (2000), incorporated by reference herein in its entirety.) Exemplary Klf family members include, Klf1, Klf2, Klf3, Klf-4, Klf5, Klf6, Klf7, Klf8, Klf9, Klf10, Klf11, Klf12, Klf13, Klf14, Klf15, Klf16, and Klf17. Klf2 and Klf-4 were found to be factors capable of generating iPS cells in mice, and related genes Klf1 and Klf5 did as well, although with reduced efficiency. (See, Nakagawa, et al., Nature Biotechnology 26:101-106 (2007), incorporated by reference herein in its entirety.) In some embodiments, variants have at least 85%, 90%, or 95% amino acid sequence identity across their whole sequence compared to a naturally occurring Klf polypeptide family member such as to those listed above or such as listed in Genbank accession number CAX16088 (mouse Klf4) or CAX14962 (human Klf4). Klf polypeptides (e.g., Klf1, Klf4, and Klf5) can be from human, mouse, rat, bovine, porcine, or other animals. Generally, the same species of protein will be used with the species of cells being manipulated. The Klf polypeptide(s) can be a pluripotency factor. The expression of the Klf4 gene or polypeptide can help induce multipotency in a starting cell or a population of starting cells.

A "Myc polypeptide" refers to any of the naturally-occurring members of the Myc family. (*See, e.g.,* Adhikary, S. & Eilers, M., Nat. Rev. Mol. Cell Biol. 6:635-645 (2005), incorporated by reference herein in its entirety.) It also includes variants that maintain similar transcription factor activity when compared to the closest related naturally occurring family member (i.e. within at least 50%, 80%, or 90% activity). It further includes polypeptides comprising at least the DNA-binding domain of a naturally occurring family member, and can further comprise a transcriptional activation domain. Exemplary Myc polypeptides include, e.g., c-Myc, N-Myc and L-Myc. In some embodiments, variants have at least 85%, 90%, or 95% amino acid sequence identity across their whole sequence compared to a naturally occurring Myc polypeptide family member, such as to those listed above or such as listed in Genbank accession number CAA25015 (human Myc). Myc polypeptides (*e.g.*, c-Myc) can be from human, mouse, rat, bovine, porcine, or other animals. Generally, the same species of protein will be used with the species of cells being manipulated. The Myc polypeptide(s) can be a pluripotency factor.

A "Sox polypeptide" refers to any of the naturally-occurring members of the SRY-related HMG-box (Sox) transcription factors, characterized by the presence of the high-mobility group (HMG) domain, or variants thereof that maintain similar transcription factor activity when compared to the closest related naturally occurring family member (*i.e.* within at least 50%, 80%, or 90% activity). It also includes polypeptides comprising at least the DNA-binding domain of the naturally occurring family member, and can further comprise a transcriptional activation domain. (*See, e.g.,* Dang, D. T. et al., Int. J. Biochem. Cell Biol. 32:1103-1121 (2000), incorporated by reference herein in its entirety.) Exemplary Sox polypeptides include, e.g., Sox1, Sox-2, Sox3, Sox4, Sox5, Sox6, Sox7, Sox8, Sox9, Sox10, Sox11, Sox12, Sox13, Sox14, Sox15, Sox17, Sox18, Sox-21, and Sox30. Sox1 has been shown to yield iPS cells with a similar efficiency as Sox2, and genes Sox3, Sox15, and Sox18 have also been shown to generate iPS cells, although with somewhat less efficiency than Sox2. (*See,* Nakagawa, et al., Nature Biotechnology 26:101-106 (2007), incorporated by reference herein in its entirety.) In some embodiments, variants have at least 85%, 90%, or 95% amino acid sequence identity across their whole sequence compared to a naturally occurring Sox polypeptide family member such as to those listed above or such as listed in Genbank accession number CAA83435 (human Sox2). Sox polypeptides (*e.g.*, Sox1, Sox2, Sox3, Sox15, or Sox18) can be from human, mouse, rat, bovine, porcine, or other animals. Generally, the same species of protein will be used with the species of cells being manipulated. The Sox polypeptide(s) can be a pluripotency factor. As discussed herein, SOX2 proteins find particular use in the generation of iPSCs.

By "differentiated hypo-immunogenic pluripotent cells" or "differentiated HIP cells" or "dHIP cells" herein is meant iPS cells that have been engineered to possess hypoimmunogenicity (*e.g.* by the knock out of B2M and CIITA and the knock in of CD47) and then are differentiated into a cell type for ultimate transplantation into subjects. Thus, for example HIP cells can be differentiated into hepatocytes ("dHIP hepatocytes"), into beta-like pancreatic cells or islet organoids ("dHIP beta cells"), into endothelial cells ("dHIP endothelial cells"), etc. Paralell definitions apply to "differentiated HIP/CD64" and differentiated HIPO-/CD64 cells.

The term percent "identity," in the context of two or more nucleic acid or polypeptide sequences, refers to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection. Depending on the application, the percent "identity" can exist over a region of the sequence being compared, e.g., over a functional domain, or, alternatively, exist over the full length of the two sequences to be compared. For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Ausubel et al., infra).

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information (www.ncbi.nlm.nih.gov/).

"Inhibitors," "activators," and "modulators" affect a function or expression of a biologically-relevant molecule. The term "modulator" includes both inhibitors and activators. They may be identified using *in vitro* and *in vivo* assays for expression or activity of a target molecule.

"Inhibitors" are agents that, *e.g.,* inhibit expression or bind to target molecules or proteins. They may partially or totally block stimulation or have protease inhibitor activity. They may reduce, decrease, prevent, or delay activation, including inactivation, desensitizion, or down regulation of the activity of the described target protein. Modulators may be antagonists of the target molecule or protein.

"Activators" are agents that, *e.g.,* induce or activate the function or expression of a target molecule or protein. They may bind to, stimulate, increase, open, activate, or facilitate the target molecule activity. Activators may be agonists of the target molecule or protein.

"Homologs" are bioactive molecules that are similar to a reference molecule at the nucleotide sequence, peptide sequence, functional, or structural level. Homologs may include sequence derivatives that share a certain percent identity with the reference sequence. Thus, in one embodiment, homologous or derivative sequences share at least a 70 percent sequence identity. In a specific embodiment, homologous or derivative sequences share at least an 80 or 85 percent sequence identity. In a specific embodiment, homologous or derivative sequences share at least a 90 percent sequence identity. In a specific embodiment, homologous or derivative sequences share at least a 95 percent sequence identity. In a more specific embodiment, homologous or derivative sequences share at least an 50, 55, 60, 65, 70, 75, 85, 86, 87, 88, 89, 90, 91, 92, 93, 94, 95, 96, 97, 98, or 99 percent sequence identity. Homologous or derivative nucleic acid sequences may also be defined by their ability to remain bound to a reference nucleic acid sequence under high stringency hybridization conditions. Homologs having a structural or functional similarity to a reference molecule may be chemical derivatives of the reference molecule. Methods of detecting, generating, and screening for structural and functional homologs as well as derivatives are known in the art.

"Hybridization" generally depends on the ability of denatured DNA to reanneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, see Ausubel et al, Current Protocols in Molecular Biology, Wiley Interscience Publishers (1995), incorporated by reference herein in its entirety.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures.

"Stringent conditions" or "high stringency conditions", as defined herein, can be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50 Mm sodium phosphate buffer at Ph 6.5 with 750 Mm sodium chloride, 75 Mm sodium citrate at 42°C; or (3) overnight hybridization in a solution that employs 50% formamide, 5 x SSC (0.75 M NaCl, 0.075 M sodium citrate), 50 Mm sodium phosphate (Ph 6.8), 0.1 % sodium pyrophosphate, 5 x Denhardt's solution, sonicated salmon sperm DNA (50 µl/ml), 0.1% SDS, and 10% dextran sulfate at 42°C, with a 10 minute wash at 42°C in 0.2 x SSC (sodium chloride/sodium citrate) followed by a 10 minute high-stringency wash consisting of 0.1 x SSC containing EDTA at 55°C.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein the term "modification" refers to an alteration that physically differentiates the modified molecule from the parent molecule. In one embodiment, an amino acid change in a CD47, HSVtk, EC-CD, or iCasp9 variant polypeptide prepared according to the methods described herein differentiates it from the corresponding parent that has not been modified according to the methods described herein, such as wild-type proteins, a naturally occurring mutant proteins or another engineered protein that does not include the modifications of such variant polypeptide. In another embodiment, a variant polypeptide includes one or more modifications that differentiates the function of the variant polypeptide from the unmodified polypeptide. For example, an amino acid change in a variant polypeptide affects its receptor binding profile. In other embodiments, a variant polypeptide comprises substitution, deletion, or insertion modifications, or combinations thereof. In another embodiment, a variant polypeptide includes one or more modifications that increases its affinity for a receptor compared to the affinity of the unmodified polypeptide.

In one embodiment, a variant polypeptide includes one or more substitutions, insertions, or deletions relative to a corresponding native or parent sequence. In certain embodiments, a variant polypeptide includes 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31-40, 41 to 50, or 51 or more modifications.

By "episomal vector" herein is meant a genetic vector that can exist and replicate autonomously in the cytoplasm of a cell; e.g. it is not integrated into the genomic DNA of the host cell. A number of episomal vectors are known in the art and described below.

By "knock out" in the context of a gene means that the host cell harboring the knock out does not produce a functional protein product of the gene. As outlined herein, a knock out can result in a variety of ways, from removing all or part of the coding sequence, introducing frameshift mutations such that a functional protein is not produced (either truncated or nonsense sequence), removing or altering a regulatory component (e.g. a promoter) such that the gene is not transcribed, preventing translation through binding to mRNA, etc. Generally, the knock out is effected at the genomic DNA level, such that the cells' offspring also carry the knock out permanently.

By "knock in" in the context of a gene means that the host cell harboring the knock in has more functional protein active in the cell. As outlined herein, a knock in can be done in a variety of ways, usually by the introduction of at least one copy of a transgene (tg) encoding the protein into the cell, although this can also be done by replacing regulatory components as well, for example by adding a constitutive promoter to the endogeneous gene. In general, knock in technologies result in the integration of the extra copy of the transgene into the host cell.

VII. Cells of the Invention

The invention provides compositions and methodologies for transplanted cells to evade the innate immune system. The invention blocks PMN cell killing by reducing cytokine or chemokine attraction or activation, danger-associated molecular patterns (DAMPs, Figure 1), and pathogen-associated molecular patterns (PAMPs). In some additional aspects of the invention, the cells will be derived from induced pluripotent stem cells (IPSC), O- induced pluripotent stem cells (iPSCO-), embryonic stem cells (ESC), O-embryonic stem cells (ESCO-), hypoimmunogenic pluripotent (HIP) cells, hypoimmunogenic pluripotent O- (HIPO-) cells, or cells derived or differentiated therefrom. In other additional aspects, the transplanted cells will be pluripotent with enhanced CD16, CD32, OR CD64 expression, or cells derived therefrom.

A. Methodologies for Genetic Alterations

The invention includes methods of modifying nucleic acid sequences within cells or in cell-free conditions. Exemplary technologies include homologous recombination, knock-in, ZFNs (zinc finger nucleases), TALENs (transcription activator-like effector nucleases), CRISPR (clustered regularly interspaced short palindromic repeats)/Cas9, and other site-specific nuclease technologies. These techniques enable double-strand DNA breaks at desired locus sites. These controlled double-strand breaks promote homologous recombination at the specific locus sites. This process focuses on targeting specific sequences of nucleic acid molecules, such as chromosomes, with endonucleases that recognize and bind to the sequences and induce a double-stranded break in the nucleic acid molecule. The double-strand break is repaired either by an error-prone non-homologous end-joining (NHEJ) or by homologous recombination (HR).

As will be appreciated by those in the art, a number of different techniques can be used to engineer the pluripotent cells of the invention. In general, these techniques can be used individually or in combination. For example, in the generation of the HIP cells, CRISPR may be used to reduce the expression of active B2M and/or CIITA protein in the engineered cells, with viral techniques (e.g. lentivirus) to knock in the CD47 functionality. Also, as will be appreciated by those in the art, although one embodiment sequentially utilizes a CRISPR step to knock out B2M, followed by a CRISPR step to knock out CIITA with a final step of a lentivirus to knock in the CD47 functionality, these genes can be manipulated in different orders using different technologies.

As is discussed more fully below, transient expression of reprogramming genes is generally done to generate/induce pluripotent stem cells.

a. CRISPR Technologies

In one embodiment, the cells are manipulated using clustered regularly interspaced short palindromic repeats)/-Cas ("CRISPR") technologies as is known in the art. CRISPR can be used to generate the starting iPSCs or to generate the HIP cells from the iPSCs. There are a large number of techniques based on CRISPR, see for example Doudna and Charpentier, Science doi: 1 0.1126/science.1258096, hereby incorporated by reference. CRISPR techniques and kits are sold commercially.

b. TALEN Technologies

In some embodiments, the HIP cells of the invention are made using **T**ranscription **A**ctivator-**L**ike **E**ffector **N**ucleases (TALEN) methodologies. TALEN are restriction enzymes combined with a nuclease that can be engineered to bind to and cut practically any desired DNA sequence. TALEN kits are sold commercially.

c. Zinc Finger Technologies

In one embodiment, the cells are manipulated using Zn finger nuclease technologies. Zn finger nucleases are artificial restriction enzymes generated by fusing a zinc finger DNA-binding domain to a DNA-cleavage domain. Zinc finger domains can be engineered to target specific desired DNA sequences and this enables zinc-finger nucleases to target unique sequences within complex genomes. By taking advantage of endogenous DNA repair machinery, these reagents can be used to precisely alter the genomes of higher organisms, similar to CRISPR and TALENs.

d. Viral Based Technologies

There are a wide variety of viral techniques that can be used to generate the HIP cells of the invention (as well as for the original generation of the iPCSs), including, but not limited to, the use of retroviral vectors, lentiviral vectors, adenovirus vectors and Sendai viral vectors. Episomal vectors used in the generation of iPSCs are described below.

e. Down regulation of genes using interfering RNA

In other embodiments, genes that encode proteins used in HLA molecules are downregulated by RNAi technologies. RNA interference (RNAi) is a process where RNA molecules inhibit gene expression often by causing specific mRNA molecules to degrade. Two types of RNA molecules - microRNA (miRNA) and small interfering RNA (siRNA) - are central to RNA interference. They bind to the target mRNA molecules and either increase or decrease their activity. RNAi helps cells defend against parasitic nucleic acids such as those from viruses and transposons. RNAi also influences development.

sdRNA molecules are a class of asymmetric siRNAs comprising a guide (antisense) strand of 19-21 bases. They contain a 5' phosphate, 2'Ome or 2'F modified pyrimidines, and six phosphotioates at the 3' positions. They also contain a sense strand containing 3' conjugated sterol moieties, 2 phospotioates at the 3' position, and 2'Ome modified pyrimidines. Both strands contain 2' Ome purines with continuous stretches of unmodified purines not exceeding a length of 3. sdRNA is disclosed in U.S. Patent No. 8,796,443, incorporated herein by reference in its entirety.

For all of these technologies, well known recombinant techniques are used, to generate recombinant nucleic acids as outlined herein. In certain embodiments, the recombinant nucleic acids (either than encode a desired polypeptide, e.g. CD47, or disruption sequences) may be operably linked to one or more regulatory nucleotide sequences in an expression construct. Regulatory nucleotide sequences will generally be appropriate for the host cell and subject to be treated. Numerous types of appropriate expression vectors and suitable regulatory sequences are known in the art for a variety of host cells. Typically, the one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are also contemplated. The promoters may be either naturally occurring promoters, or hybrid promoters that combine elements of more than one promoter. An expression construct may be present in a cell on an episome, such as a plasmid, or the expression construct may be inserted in a chromosome. In a specific embodiment, the expression vector includes a selectable marker gene to allow the selection of transformed host cells. Certain embodiments include an expression vector comprising a nucleotide sequence encoding a variant polypeptide operably linked to at least one regulatory sequence. Regulatory sequence for use herein include promoters, enhancers, and other expression control elements. In certain embodiments, an expression vector is designed for the choice of the host cell to be transformed, the particular variant polypeptide desired to be expressed, the vector's copy number, the ability to control that copy number, or the expression of any other protein encoded by the vector, such as antibiotic markers.

Examples of suitable mammalian promoters include, for example, promoters from the following genes: ubiquitin/S27a promoter of the hamster (WO 97/15664), Simian vacuolating virus 40 (SV40) early promoter, adenovirus major late promoter, mouse metallothionein-I promoter, the long terminal repeat region of Rous Sarcoma Virus (RSV), mouse mammary tumor virus promoter (MMTV), Moloney murine leukemia virus Long Terminal repeat region, and the early promoter of human Cytomegalovirus (CMV). Examples of other heterologous mammalian promoters are the actin, immunoglobulin or heat shock promoter(s).

In additional embodiments, promoters for use in mammalian host cells can be obtained from the genomes of viruses such as polyoma virus, fowlpox virus (UK 2,211,504 published 5 Jul. 1989), bovine papilloma virus, avian sarcoma virus, cytomegalovirus, a retrovirus, hepatitis-B virus and Simian Virus 40 (SV40). In further embodiments, heterologous mammalian promoters are used. Examples include the actin promoter, an immunoglobulin promoter, and heat-shock promoters. The early and late promoters of SV40 are conveniently obtained as an SV40 restriction fragment which also contains the SV40 viral origin of replication. Fiers et al., Nature 273: 113-120 (1978). The immediate early promoter of the human cytomegalovirus is conveniently obtained as a HindIII E restriction fragment. Greenaway, P. J. et al., Gene 18: 355-360 (1982). The foregoing references are incorporated by reference in their entirety.

B. Generation of Pluripotent Cells

The generation of mouse and human pluripotent stem cells (generally referred to as iPSCs; miPSCs for murine cells or hiPSCs for human cells) is generally known in the art. As will be appreciated by those in the art, there are a variety of different methods for the generation of iPCSs. The original induction was done from mouse embryonic or adult fibroblasts using the viral introduction of four transcription factors, Oct3/4, Sox2, c-Myc and Klf4; *see* Takahashi and Yamanaka Cell 126:663-676 (2006), hereby incorporated by reference in its entirety and specifically for the techniques outlined therein. Since then, a number of methods have been developed; *see* Seki et al., World J. Stem Cells 7(1):116-125 (2015) for a review, and Lakshmipathy and Vermuri, editors, Methods in Molecular Biology: Pluripotent Stem Cells, Methods and Protocols, Springer 2013, both of which are hereby expressly incorporated by reference in their entirety, and in particular for the methods for generating hiPSCs (see for example Chapter 3 of the latter reference).

Generally, iPSCs are generated by the transient expression of one or more "reprogramming factors" in the host cell, usually introduced using episomal vectors. Under these conditions, small amounts of the cells are induced to become iPSCs (in general, the efficiency of this step is low, as no selection markers are used). Once the cells are "reprogrammed", and become pluripotent, they lose the episomal vector(s) and produce the factors using the endogeneous genes. This loss of the episomal vector(s) results in cells that are called "zero footprint" cells. This is desirable as the fewer genetic modifications (particularly in the genome of the host cell), the better. Thus, it is preferred that the resulting hiPSCs have no permanent genetic modifications.

As is also appreciated by those of skill in the art, the number of reprogramming factors that can be used or are used can vary. Commonly, when fewer reprogramming factors are used, the efficiency of the transformation of the cells to a pluripotent state goes down, as well as the "pluripotency", e.g. fewer reprogramming factors may result in cells that are not fully pluripotent but may only be able to differentiate into fewer cell types.

In some embodiments, a single reprogramming factor, OCT4, is used. In other embodiments, two reprogramming factors, OCT4 and KLF4, are used. In other embodiments, three reprogramming factors, OCT4, KLF4 and SOX2, are used. In other embodiments, four reprogramming factors, OCT4, KLF4, SOX2 and c-Myc, are used. In other embodiments, 5, 6 or 7 reprogramming factors can be used selected from SOKMNLT; SOX2, OCT4 (POU5F1), KLF4, MYC, NANOG, LIN28, and SV40L T antigen.

In general, these reprogramming factor genes are provided on episomal vectors such as are known in the art and commercially available. For example, ThermoFisher/Invitrogen sell a sendai virus reprogramming kit for zero footprint generation of hiPSCs, see catalog number A34546. ThermoFisher also sells EBNA-based systems as well, see catalog number A14703.

In addition, there are a number of commercially available hiPSC lines available; *see, e.g.*, the Gibco® Episomal hiPSC line, K18945, which is a zero footprint, viral-integration-free human iPSC cell line (*see also* Burridge et al, 2011, supra).

In general, as is known in the art, iPSCs are made from non-pluripotent cells such as CD34+ cord blood cells, fibroblasts, *etc.,* by transiently expressing the reprogramming factors as described herein.

For example, successful iPSCs were also generated using only Oct3/4, Sox2 and Klf4, while omitting the C-Myc, although with reduced reprogramming efficiency.

In general, iPSCs are characterized by the expression of certain factors that include KLF4, Nanog, OCT4, SOX2, ESRRB, TBX3, c-Myc and TCL1. New or increased expression of these factors for purposes of the invention may be via induction or modulation of an endogenous locus or from expression from a transgene.

For example, murine iPSCs can be generated using the methods of Diecke et al, Sci Rep. 2015, Jan. 28;5:8081 (doi:10.1038/srep08081), hereby incorporated by reference in its entirety and specifically for the methods and reagents for the generation of the miPSCs. *See also, e.g.,* Burridge et al., PLoS One, 2011 6(4):18293, hereby incorporated by reference in its entirety and specifically for the methods outlined therein.

In some cases, the pluripotency of the cells is measured or confirmed as outlined herein, for example by assaying for reprogramming factors or by conducting differentiation reactions as outlined herein and in the Examples.

C. Generation of Hypo-Immunogenic Pluripotent (HIP) Cells

Generating HIP cells from pluripotent cells is done with as few as three genetic changes, resulting in minimal disruption of cellular activity but conferring immunosilencing to the cells.

As discussed herein, one embodiment utilizes a reduction or elimination in the protein activity of MHC I and II (HLA I and II when the cells are human). This can be done by altering genes encoding their component. In one embodiment, the coding region or regulatory sequences of the gene are disrupted using CRISPR. In another embodiment, gene translation is reduced using interfering RNA technologies. The third change is a change in a gene that regulates susceptibility to macrophage phagocytosis, such as CD47, and this is generally a "knock in" of a gene using viral technologies.

In some cases, where CRISPR is being used for the genetic modifications, hiPSC cells that contain a Cas9 construct that enable high efficiency editing of the cell line can be used; *see, e.g.*, the Human Episomal Cas9 iPSC cell line, A33124, from Life Technologies.

1. HLA-I Reduction

The HIP cells of the invention include a reduction in MHC I function (HLA I when the cells are derived from human cells).

As will be appreciated by those in the art, the reduction in function can be accomplished in a number of ways, including removing nucleic acid sequences from a gene, interrupting the sequence with other sequences, or altering the regulatory components of the nucleic acid. For example, all or part of a coding region of the gene of interest can be removed or replaced with "nonsense" sequences, frameshift mutations can be made, all or part of a regulatory sequence such as a promoter can be removed or replaced, translation initiation sequences can be removed or replaced, etc.

As will be appreciated by those in the art, the successful reduction of the MHC I function (HLA I when the cells are derived from human cells) in the pluripotent cells can be measured using techniques known in the art and as described below; for example, FACS techniques using labeled antibodies that bind the HLA complex; for example, using commercially available HLA-A,B,C antibodies that bind to the the alpha chain of the human major histocompatibility HLA Class I antigens.

a. B2M Alteration

In one embodiment, the reduction in HLA-I activity is done by disrupting the expression of the β-2 microglobulin gene in the pluripotent stem cell, the human sequence of which is disclosed herein. This alteration is generally referred to herein as a gene "knock out", and in the HIP cells of the invention it is done on both alleles in the host cell. Generally the techniques to do both disruptions is the same.

A particularly useful embodiment uses CRISPR technology to disrupt the gene. In some cases, CRISPR technology is used to introduce small deletions/insertions into the coding region of the gene, such that no functional protein is produced, often the result of frameshift mutations that result in the generation of stop codons such that truncated, non-functional proteins are made.

Accordingly, a useful technique is to use CRISPR sequences designed to target the coding sequence of the B2M gene in mouse or the B2M gene in human. After gene editing, the transfected iPSC cultures are dissociated to single cells. Single cells are expanded to full-size colonies and tested for CRISPR edit by screening for presence of aberrant sequence from the CRISPR cleavage site. Clones with deletions in both alleles are picked. Such clones did not express B2M as demonstrated by PCR and did not express HLA-I as demonstrated by FACS analysis (see examples 1 and 6, for example).

Assays to test whether the B2M gene has been inactivated are known and described herein. In one embodiment, the assay is a Western blot of cells lysates probed with antibodies to the B2M protein. In another embodiment, reverse transcriptase polymerase chain reactions (rt-PCR) confirms the presence of the inactivating alteration.

In addition, the cells can be tested to confirm that the HLA I complex is not expressed on the cell surface. This may be assayed by FACS analysis using antibodies to one or more HLA cell surface components as discussed above.

It is noteworthy that others have had poor results when trying to silence the B2M genes at both alleles. *See, e.g.* Gornalusse et al., Nature Biotech. Doi/10.1038/nbt.3860).

2. HLA-II Reduction

In addition to a reduction in HLA I, the HIP cells of the invention also lack MHC II function (HLA II when the cells are derived from human cells).

As will be appreciated by those in the art, the reduction in function can be accomplished in a number of ways, including removing nucleic acid sequences from a gene, adding nucleic acid sequences to a gene, disrupting the reading frame, interrupting the sequence with other sequences, or altering the regulatory components of the nucleic acid. In one embodiment, all or part of a coding region of the gene of interest can be removed or replaced with "nonsense" sequences. In another embodiment, regulatory sequences such as a promoter can be removed or replaced, translation initiation sequences can be removed or replaced, etc.

The successful reduction of the MHC II function (HLA II when the cells are derived from human cells) in the pluripotent cells or their derivatives can be measured using techniques known in the art such as Western blotting using antibodies to the protein, FACS techniques, rt-PCR techniques, etc.

a. CIITA Alteration

In one embodiment, the reduction in HLA-II activity is done by disrupting the expression of the CIITA gene in the pluripotent stem cell, the human sequence of which is shown herein. This alteration is generally referred to herein as a gene "knock out", and in the HIP cells of the invention it is done on both alleles in the host cell.

Assays to test whether the CIITA gene has been inactivated are known and described herein. In one embodiment, the assay is a Western blot of cells lysates probed with antibodies to the CIITA protein. In another embodiment, reverse transcriptase polymerase chain reactions (rt-PCR) confirms the presence of the inactivating alteration.

In addition, the cells can be tested to confirm that the HLA II complex is not expressed on the cell surface. Again, this assay is done as is known in the art. Exemplary analyses include Western Blots or FACS analysis using commercial antibodies that bind to human HLA Class II HLA-DR, DP and most DQ antigens as outlined below.

A particularly useful embodiment uses CRISPR technology to disrupt the CIITA gene. CRISPRs were designed to target the coding sequence of the Ciita gene in mouse or the CIITA gene in human, an essential transcription factor for all MHC II molecules. After gene editing, the transfected iPSC cultures were dissociated into single cells. They were expanded to full-size colonies and tested for successful CRISPR editing by screening for the presence of an aberrant sequence from the CRISPR cleavage site. Clones with deletions did not express CIITA as determined by PCR and did not express MHC II/ HLA-II as determined by FACS analysis.

3. Phagocytosis Reduction

In addition to the reduction of HLA I and II (or MHC I and II), generally using B2M and CIITA knock-outs, the HIP cells of the invention have a reduced susceptibility to macrophage phagocytosis and NK cell killing. The resulting HIP cells "escape" the immune macrophage and innate pathways due to one or more CD47 transgenes.

a. CD47 Increase

In some embodiments, reduced macrophage phagocytosis and NK cell killing susceptibility results from increased CD47 on the HIP cell surface. This is done in several ways as will be appreciated by those in the art using "knock in" or transgenic technologies. In some cases, increased CD47 expression results from one or more CD47 transgene.

Accordingly, in some embodiments, one or more copies of a CD47 gene is added to the HIP cells under control of an inducible or constitutive promoter, with the latter being preferred. In some embodiments, a lentiviral construct is employed as described herein or known in the art. CD47 genes may integrate into the genome of the host cell under the control of a suitable promoter as is known in the art.

The HIP cell lines were generated from B2M-/- CIITA-/- iPSCs. Cells containing lentivirus vectors expressing CD47 were selected using a Blasticidin marker. The CD47 gene sequence was synthesized and the DNA was cloned into the plasmid Lentivirus pLenti6/V5 with a blasticidin resistance (Thermo Fisher Scientific, Waltham, MA)

In some embodiments, the expression of the CD47 gene can be increased by altering the regulatory sequences of the endogenous CD47 gene, for example, by exchanging the endogenous promoter for a constitutive promoter or for a different inducible promoter. This can generally be done using known techniques such as CRISPR.

Once altered, the presence of sufficient CD47 expression can be assayed using known techniques such as those described in the Examples, such as Western blots, ELISA assays or FACS assays using anti-CD47 antibodies. In general, "sufficiency" in this context means an increase in the expression of CD47 on the HIP cell surface that silences NK cell killing. The natural expression levels on cells is too low to protect them from NK cell lysis once their MHC I is removed.

4. Suicide Genes

In some embodiments, the invention provides hypoimmunogenic pluripotent cells that comprise a "suicide gene" or "suicide switch". These are incorporated to function as a "safety switch" that can cause the death of the hypoimmunogenic pluripotent cells should they grow and divide in an undesired manner. The "suicide gene" ablation approach includes a suicide gene in a gene transfer vector encoding a protein that results in cell killing only when activated by a specific compound. A suicide gene may encode an enzyme that selectively converts a nontoxic compound into highly toxic metabolites. The result is specifically eliminating cells expressing the enzyme. In some embodiments, the suicide gene is the herpesvirus thymidine kinase (HSV-tk) gene and the trigger is ganciclovir. In other embodiments, the suicide gene is the Escherichia coli cytosine deaminase (EC-CD) gene and the trigger is 5-fluorocytosine (5-FC) (Barese et al., Mol. Therap. 20(10):1932-1943 (2012), Xu et al., Cell Res. 8:73-8 (1998), both incorporated herein by reference in their entirety.)

In other embodiments, the suicide gene is an inducible Caspase protein. An inducible Caspase protein comprises at least a portion of a Caspase protein capable of inducing apoptosis. In one embodiment, the portion of the Caspase protein is exemplified in SEQ ID NO:6. In preferred embodiments, the inducible Caspase protein is iCasp9. It comprises the sequence of the human FK506-binding protein, FKBP12, with an F36V mutation, connected through a series of amino acids to the gene encoding human caspase 9. FKBP12-F36V binds with high affinity to a small-molecule dimerizing agent, AP1903. Thus, the suicide function of iCasp9 in the instant invention is triggered by the administration of a chemical inducer of dimerization (CID). In some embodiments, the CID is the small molecule drug AP1903. Dimerization causes the rapid induction of apoptosis. (*See* WO2011146862; Stasi et al, N. Engl. J. Med 365;18 (2011); Tey et al., Biol. Blood Marrow Transplant. 13:913-924 (2007), each of which are incorporated by reference herein in their entirety.)

5. CD16, CD32, or CD64 Expression

In some aspects of the invention, the cells have a reduced susceptibility to ADCC and CDC resulting from increased CD16, CD32, or CD64 expression. The resulting cells sequester antibodies due to the increased expression. In one embodiment, the cells comprise one or more CD16, CD32, or CD64 transgenes.

a. CD16, CD32, or CD64 Increase

In some aspects, reduced ADCC or CDC susceptibility results from increased CD16, CD32, or CD64 on the cell surface. This is done in several ways as will be appreciated by those in the art using "knock in" or transgenic technologies. In some cases, increased CD16, CD32, or CD64 expression results from one or more transgenes.

Accordingly, in some embodiments, one or more copies of a CD16, CD32, or CD64 gene is added to the cells under control of an inducible or constitutive promoter, with the latter being preferred. In some embodiments, a lentiviral construct is employed as described herein or known in the art. The genes may integrate into the genome of the host cell under the control of a suitable promoter as is known in the art.

Cells containing lentivirus vectors expressing CD16, CD32, or CD64 are selected using a Blasticidin marker. The gene sequence is synthesized and the DNA may be cloned, for instance, into the plasmid Lentivirus pLenti6/V5 with a blasticidin resistance (Thermo Fisher Scientific, Waltham, MA)

In some embodiments, the expression of the gene can be increased by altering the regulatory sequences of the endogenous CD16, CD32, or CD64 gene, for example, by exchanging the endogenous promoter for a constitutive promoter or for a different inducible promoter. This can generally be done using known techniques such as CRISPR.

Once altered, the presence of sufficient expression can be assayed using known techniques such as those described in the Examples, such as Western blots, ELISA assays or FACS assays using anti-CD16, CD32, or CD64 antibodies. In general, "sufficiency" in this context means an increase in expression the cell surface that sequesters antibodies and inhibis ADCC or CDC.

6. Assays for HIP Phenotypes and Retention of Pluripotency

Once the HIP cells have been generated, they may be assayed for their hypo-immunogenicity and/or retention of pluripotency as is generally described herein and in the examples.

For example, hypo-immunogenicity are assayed using a number of techniques One exemplary technique includes transplantation into allogeneic hosts and monitoring for HIP cell growth (*e.g.* teratomas) that escape the host immune system. HIP derivatives are transduced to express luciferase and can then followed using bioluminescence imaging. Similarly, the T cell and/or B cell response of the host animal to the HIP cells are tested to confirm that the HIP cells do not cause an immune reaction in the host animal. T cell function is assessed by Elispot, Elisa, FACS, PCR, or mass cytometry (CYTOF). B cell response or antibody response is assessed using FACS or luminex. Additionally, or alternatively, the cells may be assayed for their ability to avoid innate immune responses, *e.g.* NK cell killing. NK cell lytolytic activity is assessed *in vitro* or *in vivo* using techniques known in the art.

Similarly, the retention of pluripotency is tested in a number of ways. In one embodiment, pluripotency is assayed by the expression of certain pluripotency-specific factors as generally described herein. Additionally or alternatively, the HIP cells are differentiated into one or more cell types as an indication of pluripotency.

## D. Generation of HIPO- CD16, CD32, or CD64-overexpressing Cells

In some aspects of the invention, the HIP cells generated as above will already be HIPO- cells because the process will have started with pluripotent cells having an O-blood type.

Other aspects of the invention involve the enzymatic conversion of A and B antigens. In preferred aspects, the B antigen is converted to O using an enzyme. In more preferred aspects, the enzyme is an $\alpha$-galactosidase. This enzyme eliminates the terminal galactose residue of the B antigen. Other aspects of the invention involve the enzymatic conversion of A antigen to O. In preferred aspects, the A antigen is converted to O using an $\alpha$-N-acetylgalactosaminidase. Enzymatic conversion is discussed, *e.g.*, in Olsson et al., Transfusion Clinique et Biologique 11:33-39 (2004); U.S. Pat. Nos. 4,427,777, 5,606,042, 5,633,130, 5,731,426, 6,184,017, 4, 609,627, and 5,606,042; and Int'l Pub. No. WO9923210, each of which are incorporated by reference herein in their entirety.

Other embodiments of the invention involve genetically engineering the cells by knocking out the ABO gene Exon 7 or silencing the SLC14A1 (JK) gene. Other embodiments of the invention involve knocking out the C and E antigens of the Rh blood group system (RH), K in the Kell system (KEL), Fya and Fy3 in the Duffy system (FY), Jkb in the Kidd system (JK), or U and S in the MNS blood group system. Any knockout methodology known in the art or described herein, such as CRISPR, talens, or homologous recombination, may be employed.

## E. Preferred Embodiments of the Invention

The HIP, HIPO-, iPSC, iPSCO-, ESC, or ESCO- cells, including those that overexpress CD16, CD32, or CD64, or derivatives thereof, may be used to treat, for example, Type 1 diabetes, cardiac diseases, neurological diseases, cancer, blindness, vascular diseases, and others that respond to regenerative medicine therapies. In particular, the invention contemplates using the cells for differentiation into any cell type. Particular aspects are described below:

In one aspect, the cells of the present invention comprise a nucleic acid encoding a chimeric antigen receptor (CAR). The CAR can comprise an extracellular domain, a transmembrane domain, and an intracellular signaling domain. In othe aspects, the CAR cell is derived from a HIP cell, a HIPO- cell, or overexpresses CD16, CD32, or CD64.

In some embodiments, the extracellular domain binds to an antigen selected from the group consisting of CD19, CD20, CD22, CD38, CD123, CS1, CD171, BCMA, MUC16, ROR1, and WT1. In certain embodiments, the extracellular domain comprises a single chain variable fragment (scFv). In some embodiments, the transmembrane domain comprises CD3$\zeta$, CD4, CD8$\alpha$, CD28, 4-1BB, OX40, ICOS, CTLA-4, PD-1, LAG-3, and BTLA. In certain embodiments, the intracellular signaling domain comprises CD3$\zeta$, CD28, 4-1BB, OX40, ICOS, CTLA-4, PD-1, LAG-3, and BTLA.

In certain embodiments, the CAR comprises an anti-CD19 scFv domain, a CD28 transmembrane domain, and a CD3 zeta signaling intracellular domain. In some embodiments, the CAR comprises anti-CD19 scFv domain, a CD28 transmembrane domain, a 4-1BB signaling intracellular domain, and a CD3 zeta signaling intracellular domain.

In another aspect of the invention, provided is an isolated CAR-T overexpressing cell CD16, CD32, or CD64 produced by *in vitro* differentiation of any one of the cells described herein. In some embodiments, the cell is a cytotoxic hypoimmune CAR-T cell.

In various embodiments, the *in vitro* differentiation comprises culturing the cell carrying a CAR construct in a culture media comprising one or more growth factors or cytokines selected from the group consisting of bFGF, EPO, Flt3L, IGF, IL-3, IL-6, IL-15, GM-CSF, SCF, and VEGF. In some embodiments, the culture media further comprises one or more selected from the group consisting of a BMP activator, a GSK3 inhibitor, a ROCK inhibitor, a TGF$\beta$ receptor/ALK inhibitor, and a NOTCH activator.

In particular embodiments, the isolated CAR-T cell of the invention produced by *in vitro* differentiation is used as a treatment of cancer.

In another aspect of the invention, provided is a method of treating a patient with cancer by administering a composition comprising a therapeutically effective amount of any of the isolated CAR-T cells described herein in comnbination with a PMN-inhibitory strategy. In some embodiments, the composition further comprises a therapeutically effective carrier.

In some embodiments, the administration step comprises intravenous administration, subcutaneous administration, intranodal administration, intratumoral administration, intrathecal administration, intrapleural administration, and intraperitoneal administration. In certain instances, the administration further comprises a bolus or by continuous perfusion.

In some embodiments, the cancer is a blood cancer selected from the group consisting of leukemia, lymphoma, and myeloma. In various embodiments, the cancer is a solid tumor cancer or a liquid tumor cancer.

In another aspect, the present invention provides a method of making any one of the isolated CAR-T derived from a pluripotent cell including a HIP cell, a HIPO- cell, or one that overexpresses CD16, CD32, or CD64 as described herein. The method includes *in vitro* differentiating of any one of the cells of the invention wherein *in vitro* differentiating comprises culturing them in a culture media comprising one or more growth factors or cytokines selected from the group consisting of bFGF, EPO, Flt3L, IGF, IL-2, IL-3, IL-6, IL-7, IL-15, GM-CSF, SCF, and VEGF. In some embodiments, the culture media further comprises one or more selected from the group consisting of a BMP activator, a GSK3 inhibitor, a ROCK inhibitor, a TGFβ receptor/ALK inhibitor, and a NOTCH activator.

In some aspects, the *in vitro* differentiating comprises culturing the pluripotent cells on feeder cells. In various embodiments, the *in vitro* differentiating comprises culturing in simulated microgravity. In certain instances, the culturing in simulated microgravity is for at least 72 hours.

In some aspects, provided herein is an isolated, therapeutic cardiac cell that is administered with a strategy for reducing PMN killing. In other aspects, provided herein is a method of treating a patient suffering from a heart condition or disease with a therapeutic cardiac cell and a strategy for reducing PMN killing. The method comprises administering a composition comprising a therapeutically effective amount of a population of any one of the isolated cardiac cells derived from cells of the invention as described herein in combination with a PMN inhibiting strategy. In some aspects, a PMN cell receptor is targeted. In other aspects, the cardiac cell has an elevated level of CD200 or CD99 expression. The composition further comprises a therapeutically effective carrier.

In some embodiments, the administration comprises implantation into the patient's heart tissue, intravenous injection, intraarterial injection, intracoronary injection, intramuscular injection, intraperitoneal injection, intramyocardial injection, trans-endocardial injection, trans-epicardial injection, or infusion.

In some embodiments, the heart condition or disease is selected from the group consisting of pediatric cardiomyopathy, age-related cardiomyopathy, dilated cardiomyopathy, hypertrophic cardiomyopathy, restrictive cardiomyopathy, chronic ischemic cardiomyopathy, peripartum cardiomyopathy, inflammatory cardiomyopathy, other cardiomyopathy, myocarditis, myocardial ischemic reperfusion injury, ventricular dysfunction, heart failure, congestive heart failure, coronary artery disease, end stage heart disease, atherosclerosis, ischemia, hypertension, restenosis, angina pectoris, rheumatic heart, arterial inflammation, or cardiovascular disease.

In some aspects, provided herein is a method of using a population of hypoimmune cardiac cells from a population of HIP, HIPO-, or HIPO-/CD16, CD32, or CD64 cells by *in vitro* differentiation, wherein endogenous β-2 microglobulin (B2M) gene activity and endogenous class II transactivator (CIITA) gene activity have been eliminated and CD47 expression has been increased in the HIPO-cells. The method comprises: (a) culturing a population of HIPO-cells in a culture medium comprising a GSK inhibitor; (b) culturing the population of HIPO-cells in a culture medium comprising a WNT antagonist to produce a population of pre-cardiac cells; and (c) culturing the population of pre-cardiac cells in a culture medium comprising insulin to produce a population of hypoimmune cardiac cells.

In some embodiments, the GSK inhibitor is CHIR-99021, a derivative thereof, or a variant thereof. In some instances, the GSK inhibitor is at a concentration ranging from about 2 μM to about 10 μM. In some embodiments, the WNT antagonist is IWR1, a derivative thereof, or a variant thereof. In some instances, the WNT antagonist is at a concentration ranging from about 2 μM to about 10 μM.

Some aspects of the invention utilize a therapeutic endothelial cell in combination with a PMN-inhibitory strategy. In some aspects, the endothelia cell is derived from a HIP cell, a HIPO- cell, or overexpresses CD16, CD32, or CD64. In other aspects, the therapeutic endothelial cell of the invention is selected from the group consisting of a capillary endothelial cell, vascular endothelial cell, aortic endothelial cell, brain endothelial cell, and renal endothelial cell.

In some aspects, provided herein is a method of treating a patient suffering from a vascular condition or disease. In some embodiments, the method comprises administering a composition comprising a therapeutically effective amount of a population of isolated, engineered endothelial cells of the invention in combination with a PMN-inhibitory strategy. In some embodiments, the composition further comprises a therapeutically effective carrier or excipient. In some embodiments, the administration comprises implantation into the patient's heart tissue, intravenous injection, intraarterial injection, intracoronary injection, intramuscular injection, intraperitoneal injection, intramyocardial injection, trans-endocardial injection, trans-epicardial injection, or infusion.

In some embodiments, the vascular condition or disease is selected from the group consisting of, vascular injury, cardiovascular disease, vascular disease, ischemic disease, myocardial infarction, congestive heart failure, hypertension, ischemic tissue injury, limb ischemia, stroke, neuropathy, and cerebrovascular disease.

In some aspects, provided herein is a method of producing a population of hypoimmune endothelial cells from a population of HIP, HIPO-, iPSC, iPSCO-, ESC, or ESCO- cells, including those that overexpress CD16, CD32, or CD64, or derivatives thereof. The method comprises: (a) culturing a population of HIPO-cells in a first culture medium comprising a GSK inhibitor; (b) culturing the population of HIPO-cells in a second culture medium comprising VEGF and bFGF to produce a population of pre-endothelial cells; and (c) culturing the population of pre-endothelial cells in a third culture medium comprising a ROCK inhibitor and an ALK inhibitor to produce a population of hypoimmune endothelial cells.

In some embodiments, the GSK inhibitor is CHIR-99021, a derivative thereof, or a variant thereof. In some instances, the GSK inhibitor is at a concentration ranging from about 1 $\mu$M to about 10 $\mu$M. In some embodiments, the ROCK inhibitor is Y-27632, a derivative thereof, or a variant thereof. In some instances, the ROCK inhibitor is at a concentration ranging from about 1 $\mu$M to about 20 $\mu$M. In some embodiments, the ALK inhibitor is SB-431542, a derivative thereof, or a variant thereof. In some instances, the ALK inhibitor is at a concentration ranging from about 0.5 $\mu$M to about 10 $\mu$M.

In some embodiments, the first culture medium comprises from 2 $\mu$M to about 10 $\mu$M of CHIR-99021. In some embodiments, the second culture medium comprises 50 ng/ml VEGF and 10 ng/ml bFGF. In other embodiments, the second culture medium further comprises Y-27632 and SB-431542. In various embodiments, the third culture medium comprises 10 $\mu$M Y-27632 and 1 $\mu$M SB-431542. In certain embodiments, the third culture medium further comprises VEGF and bFGF. In particular instances, the first culture medium and/or the second medium is absent of insulin.

Some aspects of the invention utilize a dopaminergic neuron (DN) in combination with a PMN-inhibitory strategy. In other aspects, the DN cell is derived from a HIP cell, a HIPO- cell, or overexpresses CD16, CD32, or CD64.

In some aspects, the isolated dopaminergic neuron is selected from the group consisting of a neuronal stem cell, neuronal progenitor cell, immature dopaminergic neuron, and mature dopaminergic neuron.

In some aspects, provided herein is a method of treating a patient suffering from a neurodegenerative disease or condition. In some embodiments, the method comprises administering a composition comprising a therapeutically effective amount of a population of DN cells and utilizing a PMN-inhibitory strategy. In some aspects, the composition further comprises a therapeutically effective carrier. In some aspects, the population of the isolated hypoimmune dopaminergic neurons is on a biodegradable scaffold. The administration may comprise transplantation or injection. In some aspects, the neurodegenerative disease or condition is selected from the group consisting of Parkinson's disease, Huntington disease, and multiple sclerosis.

In some aspects, the invention provides a method of producing a population of the DN cells by *in vitro* differentiation. In some embodiments, the endogenous $\beta$-2 microglobulin (B2M) gene activity and endogenous class II transactivator (CIITA) gene activity have been eliminated, CD47 expression has been increased, the blood group is O and Rh-, or CD16, CD32, or CD64 is overexpressed. In some aspects, the method comprises (a) culturing the population of cells in a first culture medium comprising one or more factors selected from the group consisting of sonic hedgehog (SHH), BDNF, EGF, bFGF, FGF8, WNT1, retinoic acid, a GSK3$\beta$ inhibitor, an ALK inhibitor, and a ROCK inhibitor to produce a population of immature dopaminergic neurons; and (b) culturing the population of immature dopaminergic neurons in a second culture medium that is different than the first culture medium to produce a population of dopaminergic neurons.

In some embodiments, the GSK$\beta$ inhibitor is CHIR-99021, a derivative thereof, or a variant thereof. In some instances, the GSK$\beta$ inhibitor is at a concentration ranging from about 2 $\mu$M to about 10 $\mu$M. In some embodiments, the ALK inhibitor is SB-431542, a derivative thereof, or a variant thereof. In some instances, the ALK inhibitor is at a concentration ranging from about 1 $\mu$M to about 10 $\mu$M. In some embodiments, the first culture medium and/or second culture medium are absent of animal serum.

In some aspects of the invention, the method also comprises isolating the population of DN cells from non-DN cells. In some apsects, the method further comprises cryopreserving the isolated population of hypoimmune dopaminergic neurons.

Some aspects of the invention utilize an isolated engineered hypoimmune pancreatic islet cell in combination with a PMN-inhibitory strategy. In other aspects, the pancreatic islet cell is derived from a HIP cell, a HIPO- cell, or overexpresses CD16, CD32, or CD64.

In some embodiments, the isolated hypoimmune pancreatic islet cell is selected from the group consisting of a pancreatic islet progenitor cell, immature pancreatic islet cell, and mature pancreatic islet cell.

In some aspects, provided herein is a method of treating a patient suffering from diabetes. The method comprises administering a composition comprising a therapeutically effective amount of a population of any one of the pancreatic islet cells described herein in combination with a PMN-inhibitory strategy. In some embodiments, the composition further comprises a therapeutically effective carrier. In some embodiments, the population of the isolated hypoimmune pancreatic islet cells is on a biodegradable scaffold. In some instances, the administration comprises transplantation or injection.

In some aspects, provided herein is a method of producing a population of hypoimmune pancreatic islet cells from a population of CD16, CD32, or CD64-overexpressing cells by *in vitro* differentiation. In some embodiments, the endogenous $\beta$-2 microglobulin (B2M) gene activity and endogenous class II transactivator (CIITA) gene activity have been eliminated, CD47 expression has been increased, the blood type is O and Rh- in the HIPO- cells. The method comprises: (a) culturing the population of CD16, CD32, or CD64-overexpressing cells in a first culture medium comprising one or more factors selected from the group consisting insulin-like growth factor (IGF), transforming growth factor (TGF), fibroblast growth factor (EGF), epidermal growth factor (EGF), hepatocyte growth factor (HGF), sonic hedgehog (SHH), and vascular endothelial growth factor (VEGF), transforming growth factor-β (TGFβ) superfamily, bone morphogenic protein-2 (BMP2), bone morphogenic protein-7 (BMP7), a GSK3β inhibitor, an ALK inhibitor, a BMP type 1 receptor inhibitor, and retinoic acid to produce a population of immature pancreatic islet cells; and (b) culturing the population of immature pancreatic islet cells in a second culture medium that is different than the first culture medium to produce a population of hypoimmune pancreatic islet cells.

In some embodiments, the GSK inhibitor is CHIR-99021, a derivative thereof, or a variant thereof. In some instances, the GSK inhibitor is at a concentration ranging from about 2 μM to about 10 μM. In some embodiments, the ALK inhibitor is SB-431542, a derivative thereof, or a variant thereof. In some instances, the ALK inhibitor is at a concentration ranging from about 1 μM to about 10 μM. In some embodiments, the first culture medium and/or second culture medium are absent of animal serum.

In some embodiments, the method also comprises isolating the pancreatic islet cells from non-pancreatic islet cells. In some embodiments, the method further comprises cryopreserving the isolated population of hypoimmune pancreatic islet cells.

In some aspects, provided herein is an isolated, engineered hypoimmune retinal pigmented epithelium (RPE) cell in combination with a PMN-inhibitory strategy. In other aspects, the RPE cell differentiated from a HIP cell, a HIPO- cell, or overexpresses CD16, CD32, or CD64.

In some embodiments, the isolated hypoimmune RPE cell is selected from the group consisting of a RPE progenitor cell, immature RPE cell, mature RPE cell, and functional RPE cell.

In some aspects, provided herein is a method of treating a patient suffering from an ocular condition. The method comprises administering a composition comprising a therapeutically effective amount of a population of any one of a population of the isolated RPE cells described herein in combination with a PMN-inhibitory strategy. In some embodiments, the composition further comprises a therapeutically effective carrier. In some embodiments, the population of the isolated hypoimmune RPE cells is on a biodegradable scaffold. In some embodiments, the administration comprises transplantation or injection to the patient's retina. In some embodiments, the ocular condition is selected from the group consisting of wet macular degeneration, dry macular degeneration, juvenile macular degeneration, Leber's Congenital Ameurosis, retinitis pigmentosa, and retinal detachment.

In some aspects, provided herein is a method of producing a population of RPE cells from a population of cells by in vitro differentiation. In some embodiments, the endogenous β-2 microglobulin (B2M) gene activity and endogenous class II transactivator (CIITA) gene activity have been eliminated and CD47 expression has been increased in the HIPO-cells. The method comprises: (a) culturing the population of HIPO-cells in a first culture medium comprising any one of the factors selected from the group consisting of activin A, bFGF, BMP4/7, DKK1, IGF1, noggin, a BMP inhibitor, an ALK inhibitor, a ROCK inhibitor, and a VEGFR inhibitor to produce a population of pre-RPE cells; and (b) culturing the population of pre-RPE cells in a second culture medium that is different than the first culture medium to produce a population of hypoimmune RPE cells.

In some embodiments, the ALK inhibitor is SB-431542, a derivative thereof, or a variant thereof. In some instances, the ALK inhibitor is at a concentration ranging from about 2 μM to about 10 μM. In some embodiments, the ROCK inhibitor is Y-27632, a derivative thereof, or a variant thereof. In some instances, the ROCK inhibitor is at a concentration ranging from about 1 μM to about 10 μM.

In some embodiments, the first culture medium and/or second culture medium are absent of animal serum.

In some embodiments, the method further comprises isolating the population of hypoimmune RPE cells from non-RPE cells. In some embodiments, the method further comprises cryopreserving the isolated population of hypoimmune RPE cells.

In one aspect, human pluripotent stem cells (PSCs) resist ADCC or CDC by CD16, CD32, or CD64-overexpression. In some embodiments, they are hypoimmune pluripotent stem cells (hiPSC). They are rendered hypoimmunogenic by a) the disruption of the B2M gene at each allele (e.g. B2M -/-), b) the disruption of the CIITA gene at each allele (e.g. CIITA -/-), and c) by the overexpression of the CD47 gene (CD47+, e.g. through introducing one or more additional copies of the CD47 gene or activating the genomic gene). This renders the hiPSC population B2M-/- CIITA -/- CD47tg. In a preferred aspect, the cells are non-immunogenic. In another embodiment, the HIP cells are rendered non-immunogenic B2M-/- CIITA -/- CD47tg as described above but are further modified by including an inducible suicide gene that is induced to kill the cells in vivo when required. In other aspects, CD16, CD32, or CD64-overexpressing HIPO cells are created when HIP cells are rendered blood type O by by knocking out the ABO gene Exon 7 or silencing the SLC14A1 (JK) gene and the cells are rendered Rh- by knocking out the C and E antigens of the Rh blood group system (RH), K in the Kell system (KEL), Fya and Fy3 in the Duffy system (FY), Jkb in the Kidd system (JK), or U and S in the MNS blood group system.

Alpha-1 antitrypsin deficiency (A1AD) is a genetic disorder that may result in lung disease or liver disease and is caused by a mutation in the SERPINA1 gene. The deficiency in A1AT leads to an imbalance with high neutrophil elastase activity, an enzyme that can disrupt connective tissue in the lung. Individuals with A1AD may develop chronic obstructive pulmonary disease or emphysema. Treatment of advanced disease includes intravenous infusions of the A1AT protein derived from donated human plasma or lung transplantation. The invention provides a cell therapy product to replace the missing A1AT and prevent progression of the disease. In some embodiments, the A1AT therapy maintains physiologic A1AT levels and prevents morphologic and functional diseases, *e.g.* in the lungs.

Thus, the invention provides an isolated, engineered hypoimmune cell that expresses a therapeutic protein. In some aspects of the invention, that therapeutic protein is A1AT. In other aspects, the therapeutic protein, *e.g.* A1AT, is transplanted into a subject in combination with a PMN-inhibitory strategy. In other aspects, the hypoimmune cell expressing the therapeutic protein is, or is differentiated from, a HIP cell, a HIPO- cell, or overexpresses CD16, CD32, or CD64.

## F. Maintenance of Pluripotent Cells

Once generated, the pluripotent cells, such as HIP, HIPO-, or CD16, CD32, or CD64 cells, can be maintained in an undifferentiated state as is known for maintaining iPSCs. For example, HIP cells are cultured on Matrigel using culture media that prevents differentiation and maintains pluripotency.

## G. Differentiation of Pluripotent Cells

The invention provides pluripotent cells, such as HIP, HIPO-, or CD16, CD32, or CD64 cells, that are differentiated into different cell types for subsequent transplantation into subjects in combination with a PMN-inhibitory strategy. As will be appreciated by those in the art, the methods for differentiation depend on the desired cell type using known techniques. The cells are differentiated in suspension and then put into a gel matrix form, such as matrigel, gelatin, or fibrin/thrombin forms to facilitate cell survival. Differentiation is assayed as is known in the art, generally by evaluating the presence of cell-specific markers.

In some embodiments, the pluripotent cells are differentiated into hepatocytes to address loss of the hepatocyte functioning or cirrhosis of the liver. There are a number of techniques that can be used to differentiate HIPO- cells into hepatocytes; see for example Pettinato et al., doi:10.1038/spre32888, Snykers et al., Methods Mol Biol 698:305-314 (2011), Si-Tayeb et al, Hepatology 51:297-305 (2010) and Asgari et al., Stem Cell Rev (:493-504 (2013), all of which are hereby expressly incorporated by reference in their entirety and specifically for the methodologies and reagents for differentiation. Differentiation is assayed as is known in the art, generally by evaluating the presence of hepatocyte associated and/or specific markers, including, but not limited to, albumin, alpha fetoprotein, and fibrinogen. Differentiation can also be measured functionally, such as the metabolization of ammonia, LDL storage and uptake, ICG uptake and release and glycogen storage.

In some embodiments, the pluripotent cells are differentiated into beta-like cells or islet organoids for transplantation to address type I diabetes mellitus (T1DM). Cell systems are a promising way to address T1DM, *see, e.g.,* Ellis et al., doi/10.1038/nrgastro.2017.93, incorporated herein by reference. Additionally, Pagliuca *et al.* reports on the successful differentiation of β-cells from hiPSCs (*see* doi/10.106/j.cell.2014.09.040, hereby incorporated by reference in its entirety and in particular for the methods and reagents outlined there for the large-scale production of functional human β cells from human pluripotent stem cells). Furthermore, Vegas *et al.* shows the production of human β cells from human pluripotent stem cells followed by encapsulation to avoid immune rejection by the host; (doi:10.1038/nm.4030, hereby incorporated by reference in its entirety and in particular for the methods and reagents outlined there for the large-scale production of functional human β cells from human pluripotent stem cells).

Differentiation is assayed as is known in the art, generally by evaluating the presence of β cell associated or specific markers, including but not limited to, insulin. Differentiation can also be measured functionally, such as measuring glucose metabolism, see generally Muraro et al, doi:10.1016/j.cels.2016.09.002, hereby incorporated by reference in its entirety, and specifically for the biomarkers outlined there.

Once the differentiated beta cells are generated, they can be transplanted (either as a cell suspension or within a gel matrix as discussed herein) into the portal vein/liver, the omentum, the gastrointestinal mucosa, the bone marrow, a muscle, or subcutaneous pouches.

In some embodiments, the pluripotent cells are differentiated into retinal pigment epithelium (RPE) to address sight-threatening diseases of the eye. Human pluripotent stem cells have been differentiated into RPE cells using the techniques outlined in Kamao et al., Stem Cell Reports 2014:2:205-18, hereby incorporated by reference in its entirety and in particular for the methods and reagents outlined there for the differentiation techniques and reagents; *see also* Mandai *et al.,* doi:10.1056/NEJMoa1608368, also incorporated in its entirety for techniques for generating sheets of RPE cells and transplantation into patients.

Differentiation can be assayed as is known in the art, generally by evaluating the presence of RPE associated and/or specific markers or by measuring functionally. See for example Kamao et al., doi:10.1016/j.stemcr.2013.12.007, hereby incorporated by reference in its entirety and specifically for the markers outlined in the first paragraph of the results section.

In some embodiments, the pluripotent cells are differentiated into cardiomyocytes to address cardiovascular diseases. Techniques are known in the art for the differentiation of hiPSCs to cardiomyoctes and discussed in the Examples. Differentiation can be assayed as is known in the art, generally by evaluating the presence of cardiomyocyte associated or specific markers or by measuring functionally; see for example Loh et al., doi: 10.1016/j.cell.2016.06.001, hereby incorporated by reference in its entirety and specifically for the methods of differentiating stem cells including cardiomyocytes.

In some embodiments, the pluripotent cells are differentiated into endothelial colony forming cells (ECFCs) to form new blood vessels to address peripheral arterial disease. Techniques to differentiate endothelial cells are known. *See, e.g.,* Prasain *et al.,* doi: 10.1038/nbt.3048, incorporated by reference in its entirety and specifically for the methods and reagents for the generation of endothelial cells from human pluripotent stem cells, and also for transplantation techniques. Differentiation can be assayed as is known in the art, generally by evaluating the presence of endothelial cell associated or specific markers or by measuring functionally.

In some embodiments, the pluripotent cells are differentiated into thyroid progenitor cells and thyroid follicular organoids that can secrete thyroid hormones to address autoimmune thyroiditis. Techniques to differentiate thyroid cells are known the art. *See, e.g.* Kurmann *et al.,* doi:10.106/j.stem.2015.09.004, hereby expressly incorporated by reference in its entirety and specifically for the methods and reagents for the generation of thyroid cells from human pluripotent stem cells, and also for transplantation techniques. Differentiation can be assayed as is known in the art, generally by evaluating the presence of thyroid cell associated or specific markers or by measuring functionally.

H. Transplantation of Differentiated Pluripotent Cells

As will be appreciated by those in the art, the differentiated pluripotent cell derivatives are transplated using techniques known in the art that depends on both the cell type and the ultimate use of these cells. In general, the cells of the invention are transplanted either intravenously or by injection at particular locations in the patient. When transplanted at particular locations, the cells may be suspended in a gel matrix to prevent dispersion while they take hold.

In order that the invention described herein may be more fully understood, the following examples are set forth. It should be understood that these examples are for illustrative purposes only and are not to be construed as limiting this invention in any manner.

## VIII. **EXAMPLES**

HIP, HIPO-, and CD16/CD32/CD64 cells are generated as disclosed in WO2018/132783, PCT/US19/42123, PCT/US19/42117, and Prov. Appl. Nos. 62/698,973, 62/698,978, 62/698,981, 62/698,984, 62/846,399, 62/855,499, and 62/915,601 each of which are incorporated by reference herein in their entirety.

A. **Example 1: Activated Polymorphonuclear (PMN) Cells Kill Transplanted Cells**

**Allogeneic HIP cells are killed in a pro-inflammatory environment.** A comprehensive human immune assay was established using the XCelligence *in vitro* impedance system that obtains real-time kinetics of target cell killing. (ACEA BioSciences, San Diego, CA., Catalog No. 00380601040). Wild-type (wt) and engineered iPSCs were differentiated into endothelial cells for this assay. All components of the human immune system found in blood were included into an effector mix combining serum (contains antibodies, complement, and soluble factors like cytokines and growth factors), peripheral blood mononuclear cells (PBMCs, contains all lymphocytes, monocytes, and NK cells) and PMNs (contains mainly neutrophils and few basophils and eosinophils). Notably, the serum was taken from blood type AB donors and thus did not contain blood type antibodies. The detrimental effect of blood type antibodies has previously been established. Some experimental groups were treated with IL-2 (1 ng/ml), which is a central cytokine involved in allograft rejection. The assays were run for a duration of 90h which is the maximum time period iECs can be cultured in this system before the monolayer breaks down spontaneously.

Figure 3 shows that viable human wt or dKO iECs were rejected *in vitro* by an allogeneic effector mix containing all components of the immune system even in the absence of the pro-inflammatory cytokine IL-2. HIP cells were rejected only in the presence of IL-2. This points to the fact that HIP iECs are still susceptible to immune killing in a pro-inflammatory environment. The addition of IL-2 also accelerated killing of wt and dKO iECs. Thus, there still is a previously unidentified immune barrier for the transplantation of HIP cell derivatives.

The XCelligence assays were repeated without serum to evaluate contributions of soluble factors to the target cell killing. Figure 4 shows that HIP cells were killed by a mixture of allogeneic PBMCs and PMNs in the presence of the proinflammatory cytokine IL-2 with similar kinetics as shown in Figure 3. Thus, serum factors besides blood type antibodies did not affect allogeneic target cell killing if the recipient does not have preformed anti-HLA antibodies. The results conclude that HIP cells were killed by a cellular component.

Individual XCelligence assays in Figures 5A and 5B were run with purified CD3+ lymphocytes or NK cells (Figure 5A) or macrophages or PMNs (Figure 5B) to assess their individual interactions with the target cells. The assays showed that PMNs were the only cell population able to kill HIP cells, but they required inflammatory activation.

CD3+ lymphocytes killed allogeneic wt iECs but spared dKO and HIP cells; IL-2 accelerated the killing. Unstimulated NK cells exerted no killing, but IL-2 activated them to vigorously kill dKO iECs. Macrophages (Macs) needed no stimulation and very effectively killed only dKO iECs. HIP were spared by all PBMC populations. Resting PMNs did not attack any of the target cells, but IL-2 activated PMNs did kill all target cells with the same kinetics irrespective of HLA or CD47 engineering. Thus, PMNs were the only cell population that could kill the HIP cells but they required inflammatory activation.

## B. Example 2: Necrotic Cells Activate PMNs and Jeopardize Transplanted Cells

The immune system not only distinguishes between "self" and "non-self", but also reacts to "danger" signals: released by autologous cells in distress. Matzinger, Ann. Rev. Immunol. 12:991-1045 (1994), incorporated by reference herein in its entirety. Intracellular components, released by damaged cells, can act as adjuvants and boost immune activation to alarm the immune system of cell death. Because apoptosis activates programed cell death pathways aimed at digesting intracellular proteins, apoptotic cells are considered weak releasers of danger signals. Necrotic cells, however, release intracellular factors without prior modification and show more effective leakage of DAMPs. For our *in vitro* assays, 20,000 target cells were sonicated to disrupt their cell membrane and release DAMPs and the cell debris was added to prior grown and confluent target iEC layer in Xcelligence assays.

Thus, for DAMP-activated XCelligence assays, iECs were plated and in addition to the immune effector cells, 20,000 sonicated target cells were added at the same time. Figure 6 shows that necrotic cells activated PMNs that in turn killed healthy target cells as collateral damage. All wt, dKO, and HIP iECs were killed at the same pace. When PBMCs were mixed with PMNs, the killing kinetics were slower. This was attributed to the 50% reduced PMN content in the mixed immune cell population. Overall, the results show a potent innate PMN killing mechanism that is independent of target cell immune features and is activated by the presence of necrotic cells.

Since DAMPs induce PMN activation in an attempt by the immune system to contain and clear damaged sites, healthy bystander cells are often collateral damage. Minimizing cell death after transplantation will minimize the release of DAMPs.

## C. Example 3: Minimizing IL-2 Stimulation Reduces PMN Activation

Cyclosporine and dexamethasone reduced, but did not eliminate, PMN killing stimulated by IL-2 (Figure 7). Unstimulated human PMNs did not show any killing of wt, dKO, or HIP cells (Figure 5B) but IL-2 stimulation triggered killing. Thus, minimizing IL-2 release alleviated PMN activation. Cyclosporine and dexamethasone were analyzed for their efficacy to dampen a PMN killing response even after IL-2 stimulation. XCelligence assays were performed with human wt, dKO, and HIP cells against IL-2 stimulated human PMNs (Figure 7). Cyclosporine was used in two different concentrations (400ng/ml (high) and 40ng/ml (low)). Both of these compounds showed some efficacy in reducing PMN killing induced by IL-2.

## D. Example 4: Engaging PMN Inhibitory Receptors Reduce PMN Activation and Killing

PMNs have several receptors that bind phosphatidylserines, phosphatidyl-ethanolamines, sialic acids, or syalylated proteins. They are abundant and cover the cell membranes. Some receptors also bind protein ligands. Such receptors signal through inhibitory ITIM or NPXY domains and include CD200R, PIRL$\alpha$, and SIRL-1. PMN killing was reduced by engaging these receptors as shown below XCelligence assays.

Ligands for CD200R and PIRL$\alpha$ are CD200 and CD99, respectively. Recombinant human CD200 Fc chimera proteins (R&D Systems, Minneapolis, MN, Catalog no. 2724-CD-050) and CD99 Fc chimera proteins (R&D Systems, Minneapolis, MN, Catalog no. 3968-CD-050) engage these receptors. These Fc chimera proteins were added to the assays at 10$\mu$g/ml. There is no known ligand for SIRL-1 but there is an activating monoclonal SIRL-1 antibody to trigger downstream inhibitory signaling (Steevels TA., Eur J Immunol. 43(5):1297-308 (2013)). The human PMNs were activated via DAMPs or IL-2 and the killing of human wt and HIP iECs was assessed (Figure 8). Engagement of either one of those inhibitory receptors prevented PMN killing in the presence of necrotic cells (sonicated cells). No receptor pathway, however, was efficient enough to fully prevent killing by IL-2 activated human PMNs although it was somewhat delayed. Thus, inhibitory receptor engagement is partially effective to inhibit PMN killing but may need to be combined with other strategies to improve efficacy.

E. Example 5: Neutrophil Elastase Inhibitors Reduce PMN Killing

The Neutrophil Elastase (NE) inhibitor BAY 85-8501 (MedChem Express, Monmouth Junction, NJ, Catalog No. HY-19908, 1nM or 1μM for *in vitro* Xcelligence assays), is a small-molecule inhibitor of high selectivity and potency that had recently been shown safety in a human clinical trial (von Nussbaum F., Chem. Med. Chem. 10:1163-73 (2015); Watz H., Pulm. Pharmacol. Ther. 2019;56:86-93 (2019), each of which are incorporated by reference herein in their entirey). BAY 85-8501 was used at 1nM or 1μM concentrations. The effector PMNs were stimulated with sonicated cells or IL-2.

Figure 9A shows that the higher concentration of BAY 85-8501 (1μM) fully prevented human wt or HIP target cell killing when PMNs were activated via DAMPs from the necrotic (sonicated) cells. The same concentration of BAY 85-8501 further delayed the target cell killing in the presence of IL-2.

Alpha-1 antitrypsin (A1AT, Sigma-Aldrich, St. Louis, MO, Catalog No. A9024, 30μM for *in vitro* Xcelligence assays), another Neutrophil Elastase inhibitor (Polverino E. Chest. 152(2):249-262 (2017)) was minorly effective in reducing PMN-mediated killing of human wt and HIP cells (Figure 9B).

F**. Example 6: Combination of PMN Inhibitory Receptor Engagement and** Neutrophil Elastase Inhibitors

The anti-SIRL-1 antibody (MyBioSource, San Diego, CA, Catalog No. MBS584764) in combination with BAY 85-8501 was a highly-potent PMN inhibitor regimen in XCelligence assays with human PMNs activated via DAMPs or IL-2 (Figure 10).

The combination of the anti-SIRL-1 antibody and BAY 85-8501 was a tailored approach for PMN inhibition or overall immunosuppression. An XCelligence assay that combined all human immune system components PBMCs, PMNs, and serum in a pro-inflammatory IL-2 environment (Figure 11) was used. This is a comprehensive and highly-stringent general *in vitro* immune assay. The combination of BAY 85-8501and the anti-SIRL mAb was inefficient at preventing wt and dKO killing, which is known to be caused by allogeneic CD3+ lymphocytes (wt) or NK cells and Macs (dKO) from prior experiments (Figure 5A and B). The regimen is thus not generally immunosuppressive. It prevents only PMN killing as shown by the survival of HIP iECs, because those target cells are only killed by activated PMNs and killing is inhibited by anti-SIRL-1 and BAY 85-8501.

The efficacy of Cyclosprorin A (CsA) combined with BAY 85-8501 was confirmed in the presence of PBMCs and DAMP-activated PMNs using the XCelligence assay (Figure 12). Low-dose CsA (40ng/ml) was combined with BAY 85-8501, with or without the anti-SIRL-1 mAb. The combination of low-dose CsA and BAY 85-8501 alone was highly effective in preventing killing of all target cells. CsA and BAY 85-8501 are clinically tested drugs and a translational approach into the clinic would be possible. The anti-SIRL-1 mAb is a beneficial addition to this regimen but there is no clinical grade product available at this time.

G. **Example 7: PMN Killing is Diminished by Inhibiting Activating Receptors**

Activating receptors include the family of toll-like receptors (TLRs) that are activated by a wide range of ligands including DAMPs. PMN activation pathways were inhibited by TAK-242 (MedChem Express, Monmouth Junction, NJ, Catalog No. HY-11109, 10μM for *in vitro* Xcelligence assays), a small-molecule inhibitor of TLR-4-mediated signaling. Wild-type and HIP cells were used on the XCelligence platform using PMNs as effector cells (Figure 13). Necrotic cells or IL-2 were used as PMN stimulators. Inhibition of TLR-4 signaling with TAK-242 diminished PMN killing. The efficacy was stronger with the somewhat weaker stimulatory signal via DAMPs. With IL-2, PMN killing was still markedly reduced.

Figure 14 shows that PMN killing by DAMP- or IL-2 activated PMNs was mildly diminished by another inhitibor of TLR-4 signialling, Idelalisib (Idel, MedChem Express, Monmouth Junction, NJ, Catalog No. HY-13026, 10μM for *in vitro* Xcelligence assays). It was similarly effective for wt and HIP iEC target cells.

Figure 15 shows that inhibiting PMN killing by DAMP- or IL-2 activated PMNs was also diminished by CpG-52364, an inhibitor of TLR7/8/9 signaling (RayStar Biosystems, HangZhou, China, Catalog No. GY05368, 10μM for *in vitro* Xcelligence assays). It was similarly effective for wt and HIP iEC target cells.

Figure 16 shows that colchicine, an inhibitor of microtubule polymerization, was a mild inhibitor of DAMP- or IL-2 activated PMN killlling (Sigma-Aldrich, St. Louis, MO, Catalog No. C9754, 1μM for *in vitro* Xcelligence assays). It was again similarly effective for wt and HIP iEC target cells.

H. Example 8: PMN Killing is Diminished by Inhibiting the Release of Superoxide

Apocynin is a naturally occurring methoxy-substituted catechol, experimentally used as an inhibitor of NADPH-oxidase. It can decrease the production of superoxide from activated PMNs while the ability of phagocytosis remains unaffected (Stefanska J. Mediators Inflamm. 2008:106507 (2008)).

Figure 17 shows that apocynin (Apo) diminished DAMP- or IL-2 activated PMN killling (Cayman Chemical, Ann Arbor, MI, Catalog No. 11976, 10$\mu$M for *in vitro* Xcelligence assays). Apocynin reduced PMN killing for wt and HIP iEC target cells.

## I. Example 9: PMN Killing is very Effectively Diminished by Combining Agents with Different Modes of Action

Since PMNs are very complex effector cells that can be activated via numerous different activation pathways and can exert different killing mechanisms. The combination of drugs or approaches with different modes of action showed synergistic effects on PMN inhibition.

Colchicine was combined with either BAY 85-8501, TAK-242, or both to maximize the inhibitory effect on PMNs. Figure 18A shows XCelligence assays with PMNs activated via DAMPs and Figure 18B used IL-2 stimulation. All combinations very effectively and completely prevented PMN killing of wt and HIP iECs.

A multi-drug combination of idelalisib, apocynin, colchicine, BAY-85-8501, and TAK-242 in Figure 19 did not only confirm the effective inhibition of PMN killing, it also showed that such multi-drug combinations are specific for their targets and cause no toxic target cell killing.

## J. Example 10: Cell therapy for the treatment of Alpha1-Antitrypsin Deficiency

Induced endothelial cell (iEC) grafts were used as factories to replace a crucial missing factor responsible for disease in a mouse model of alpha1-antitiypsin (A1AT) deficiency that quickly develops both structural and functional changes of lung disease. In C57BL/6 (B6) mice with quintuple *Serpina1a-e* knockouts, a mild lipopolysaccharide (LPS) challenge that is well tolerated in wt mice leads to emphysema development due to the unabated activity of the secreted neutrophil elastase from recruited neutrophils.

Wild type B6 mouse induced pluripotent stem cells (iPSCs) were generated and underwent gene editing to make them hypoimmunogenic. The *B2m* and *Ciita* genes were targeted for disruption with Cas9 nuclease with guides to deplete MHC class I and class II expression. CD47 was overexpressed using lentiviral transfection. The wild type and hypoimmunogenic (HIP) iPSCs were transfected to express firefly luciferase (FLuc) and were differentiated into iPSC-derived iECs. Although the HIP iPSCs were used, the emphysema model described is syngeneic.

To turn iECs into enzyme factories, HIP iECs were engineered to secrete human A1AT. The mouse *Serpinale* cDNA sequence (Appendix) was synthesized, cloned into a lentiviral vector with zeocin resistance, HIP iPSC clones were transduced, and antibiotic selected pools of HIP$_{SERPINE1}$ iPSCs were expanded. These iPSCs were differentiated into HIP$_{SERPINE1}$ iECs and showed the classic EC phenotype of wt and HIP iECs (**Fig. 20A**). A total of $1.5 \times 10^5$ wt, HIP, and HIP$_{SERPINE1}$ iECs were plated for 24 h and A1AT levels were measured (**Fig. 20B**). The HIP$_{SERPINE1}$ iECs produced approximately 50 ng A1AT in one day.

With a half-life of A1AT in mice of approx. 4.5 days, it was estimated that a cell number of $1.5 \times 10^8$ HIP$_{SERPINE1}$ iECs was necessary to restore and maintain physiologic A1AT serum levels (about 200 $\mu$g/ml). Seven days before induction of lung disease, *Serpina* -/- mice received $1.5 \times 10^8$ HIP$_{SERPINE1}$ iECs in matrigel injected into the peritoneum and the subcutaneous tissue in the lumbar area to split the cell load (**Fig. 21a**). *Serpina*$^{-/-}$ mice without cell injection as well as healthy B6 mice served as controls. Twenty-one days later, A1AT levels in the cell therapy group were approx. 75% of those in the healthy B6 mice and thus well in the physiologic range (**Fig. 22**).

Lung disease was induced in both *Serpina*$^{-/-}$ groups with LPS injection on day 0 (7 days after cell injection) and repeated on day 11 (**Fig. 21B**). On day 14, pulmonary mechanics were assessed using a computer-controlled piston ventilator for standardized measurements. Untreated *Serpina*$^{-/-}$ mice developed ventilation patterns consistent with developing emphysema including increased lung compliance and decreased elastance (**Fig. 23).** The coefficients of tissue damping and tissue elasticity were both reduced and the total lung capacity was increased. Consistent with prior reports of this model, resistance did not change. After LPS induction, all lung function parameters remained within the range of completely healthy B6 mice. Thus, HIP$_{SERPINE1}$ iEC therapy was effective in preventing the development of respiratory patterns typical of emphysematous lung disease in *Serpina*$^{-/-}$ mice.

The lungs were fixed for histology and serially cut for stereological assessment. Untreated B6 mice are shown in (**Fig. 24**). Morphometric criteria for emphysema in untreated *Serpina*$^{-/-}$ mice including a decrease in volume fraction of alveolar septal tissue, an increase in the fraction of alveolar volume, a decrease in surface density of alveolar septa, and an increase in the mean linear intercept (**Fig. 25**). HIP$_{SERPINE1}$ iEC therapy successfully prevented all structural damages to the lung tissue (**Fig. 26**).

*Stereological lung assessment.* Histology slides were scanned and assessed in a standardized way (**Fig. 27**). Stereological lung assessment (mean $\pm$ s.d., 6 animals per group, analysis of variance (ANOVA)). Morphometric criteria for emphysema in untreated *Serpina*$^{-/-}$ mice were found. Parameters describing distal airspace enlargement and loss of alveoli and alveolar surface area (volume fraction of alveolar septal tissue, fraction of alveolar volume, surface density of alveolar septa), and increase in mean linear intercept (chord) length, a parameter to describe the mean free distance in the air spaces were present. HIP$_{Serpine1}$ iEC therapy was successful in preventing all structural damages to the lung tissue.

Since *Serpina$^{-/-}$* mice were generated on a B6 background, allogeneic BALB/c recipients were used to separately assess graft survival. All wt B6 iEC grafts were rejected over 14 days and all HIP$_{Serpina1e}$ iEC grafts showed survival over the study period (**Fig. 28**). Overall, A1AT repletion via cell therapy was able to avert the development of emphysematous lung disease in *Serpina$^{-/-}$* mice.

**Methods**

**Mice:** BALB/c (BALB/cAnNCrl, H2$^d$) and C57BL/6 (C57BL/6J, H2$^b$) (all 6-12 weeks) were purchased from The Jackson Laboratory (Sacramento, CA). *Serpina$^{-/-}$* mice were generated by Christian Mueller as reported previously. Borel et al., Proc Natl Acad Sci USA 115:2788-2793 (2018), incorporated by reference herein in its entirety. The number of animals per experimental group is presented in each figure. Mice received humane care in compliance with the Guide for the Principles of Laboratory Animals. Animal experiments were approved by the University of California San Francisco (UCSF) Institutional Animal Care and Use Committee and performed according to local guidelines.

**Mouse iPSC culture:** Mouse iPSCs were generated from tail tip fibroblasts as reported previously[2]. On confluent MEF feeder cells, iPSCs were grown in KO DMEM 10829 with 15% KO Serum Replacement, 1% glutamine, 1% MEM-NEAA, 1% pen-strep (all Gibco, Grand Island, NY), 0.2% beta-mercaptoethanol, and 100 units LIF (both Millipore, Billerica, MA). Cells were maintained in 10 cm dishes and medium was changed daily. Cells were passaged every 2-3 days using 0.05% trypsin-EDTA (Gibco). Prior to experiments, iPSCs were cultured on gelatin (Millipore, Billerica, MA) without feeders using the standard media. Cell cultures were regularly screened for mycoplasma infections using the MycoAlert Kit (Lonza, Basel, Switzerland). Gene editing was performed as described previously[2].

**Transduction to express firefly luciferase:** One hundred thousand iPSCs were plated in gelatin coated 6-well plates and incubated overnight at 37° C at 5% CO$_2$. Media was then changed and one vial of Fluc lentiviral particles expressing luciferase II gene under re-engineered EF1a promotor (GenTarget, San Diego, CA) was added to 1.5 ml media. After 36 h, 1 ml of cell media was added. After further 24 h, complete media change was performed. After 2 days, luciferase expression was confirmed by adding D-luciferin (Promega, Madison, WI). Signals were quantified with IVIS 200 (Perkin Elmer Waltham, MA) in maximum photons s $^{-1}$ cm $^{-2}$ per steradian.

**Derivation and characterization of iPSC-derived endothelial cells (iECs):** iPSC were plated on gelatin in 6-well plates and maintained in mouse iPSC media until they reached 60% confluency. To start the differentiation, media was changed to RPMI-1640 containing 2% B-27 minus Insulin (both Gibco, Grand Island, NY) and 5 μM CHIR-99021 (Selleckchem, Munich, Germany). On day 2, the media was changed to reduced media: RPMI-1640 containing 2% B-27 minus Insulin (both Gibco) and 2 μM CHIR-99021 (Selleckchem, Munich, Germany). From day 4 to day 7, cells were exposed to RPMI-1640 EC media, RPMI-1640 containing 2% B-27 minus Insulin plus 50 ng/mL mouse vascular endothelial growth factor (mVEGF; R&D Systems, Minneapolis, MN), 10 ng/mL mouse fibroblast growth factor basic (mFGFb; R&D Systems), 10 μM Y-27632 (Sigma-Aldrich, Saint Louis, MO), and 1 μM SB 431542 (Sigma-Aldrich). Endothelial cell clusters were visible from day 7 and cells were maintained in EGM-2 SingleQuots media (Lonza, Basel, Switzerland) plus 10% FCS hi (Gibco), 25 ng/mL mVEGF, 2 ng/mL mFGFb, 10 μM Y-27632 (Sigma-Aldrich), and 1 μM SB 431542. The differentiation process was completed after 21 days and undifferentiated cells detached during the differentiation process. For purification, cells went through MACS purification according the manufactures' protocol using anti-CD15 mAb-coated magnetic microbeads (Miltenyi, Auburn, CA) for negative selection. The highly purified miECs in the flow-through were cultured in EGM-2 SingleQuots media plus supplements and 10% FCS hi. TrypLE was used for passaging the cells 1:3 every 3 to 4 days. Their phenotype was confirmed by immunofluorescence (IF) for CD31 (ab28364, Abcam), and VE-cadherin (sc-6458, Santa Cruz Biotechnology, Santa Cruz, CA). Briefly, cells were fixed with 4% paraformaldehyde in PBS for 15 min. Cell membranes were permeabilized with Permeabilization solution (ASB-0102, Applied StemCell, Milpitas, CA), followed by Blocking solution (ASB- 0103, Applied StemCell) and incubation with the primary antibodies. For visualization, cells were incubated with secondary antibody conjugated with AF488 or AF555 (Invitrogen, Carlsbad, CA). After nuclei staining with DAPI, images were obtained and analyzed with a Leica SP5 laser confocal microscope (Leica, Wetzlar, Germany). For the tube formation assay, $2.5 \times 10^5$ iECs were stained with 5 μM CFSE and 0.1 μg/mL Hoechst (both Thermo Fisher, Waltham, MA) for 10 min at RT and plated on 10 mg/mL undiluted Matrigel (356231, Corning, Corning, NY) in 24-well plates. After 48 h, tube formations were visualized by IF. PCR was performed as described above. The following primers were used: VE-cadherin (Cdh5): forward: 5'-GGATGCAGAGG CTCACAGAG-3', reverse: 5'-CTGGCGGTTCACGTTGGACT-3'. All other primers were included in the Mouse ES/iPS cell Pluripotency RT-PCR Kit (Cat# ASK-6001, Applied StemCell).

**Gene editing to generate HIP$_{Serpina1e}$ iPSCs:** the *Serpina1e* gene sequence (NM_009247.2) was synthesized and cloned into a lentivirus with zeocin resistance, which was used to transduce HIP iPSCs, followed by antibiotic selection and expansion of HIP$_{Serpina1e}$ iPSCs. Clones were picked and expanded and their Serpina1e mRNA levels were quantified. Of 3 clones with stably high *Serpina1e* expression and confirmed pluripotency markers Nanog, Oct4, and Sox2, the clone with the highest *Serpinale* expression was chosen for this study.

**Graft survival by BLI:** D-luciferin firefly potassium salt (375 mg/kg; Biosynth AG, Staad, Switzerland) was dissolved in PBS (pH 7.4) (Gibco, Invitrogen) and was injected i.p. (250 μL per mouse) into anesthetized mice. Animals were imaged using the Ami HT system (Spectral Instruments Imaging). Region of interest (ROI) bioluminescence was quantified in units of maximum photons s $^{-1}$ cm $^{-2}$ per steradian. The maximum signal from an ROI was measured using Aura Image software (Spectral Instruments Imaging, Tucson, AZ).

**Mouse lung emphysema model:** B6 or *Serpina$^{-/-}$* mice received two sequential orotracheal doses of LPS to induce lung disease. Mice were anesthetized with an i.p. dose of a ketamine/xylazine mixture (90 mg/kg of ketamine and 4.5 mg/kg of xylazine) and were placed in dorsal recumbency on a rodent work stand (Braintree Scientific, Braintree, MA) and intubated. Following instillation, mice received three ventilations with 0.2 mL of air.

**FlexiVent:** Mice were anesthetized as outlined above. A tracheotomy was performed and a pre-calibrated cannula was introduced into the trachea. The mouse was then placed on a computer-controlled piston-ventilator flexiVent system (Scireq, Monteal, Canada) and ventilated at a tidal volume of 10 mL/kg, rate of 150 breaths per minute and a positive end expiratory pressure of 3 mmHg. Neuromuscular blockade with pancuronium bromide (2.5 mg/kg) was given to prevent spontaneous respiratory effort. Measurements were obtained as previously described.

**Lung stereology:** Immediately following measurements of lung mechanics (flexiVent), lungs were harvested for stereologic analysis. The chest was opened, and the pulmonary circulation flushed via right ventricular puncture with 10 mL cold PBS. The degassed lung was inflated with 1% ultra-low temperature gel agarose (Sigma-Aldrich) in neutral buffered formalin (10%) to 25 cm pressure. Once fixed, lung volume was measure by water displacement. Subsequently, lung tissues were oriented randomly in cassettes and embedded in paraffin, followed by sectioning started at random depth at a uniform thickness (10 μm). Slides were stained with hematoxylin and eosin and 10 randomly oriented nonoverlapping fields from each section were photographed. Lung morphology was quantified using the Stepanizer software (www.stepanizer.com). Surrogate markers for lung morphology were calculated as follows: Volume fraction of alveolar septal tissue:

$$Vv(sept/par) = \sum P(sept)/\sum P(par),$$

Fraction of alveolar volume:

$$Vv(alv/par) = \sum P(alv)/\sum P(par)$$

Surface density of alveolar septa:

$$Sv(sept/par) = (2 \times \sum I)/(\sum P(par) \times l/p)$$

Mean linear intercept:

$$Lm = 2 \cdot k \cdot d \cdot P(asp)/I(A).$$

### K. Example 11: PMN Inhibition Promotes Human HIP iEC$_{SERPINA1}$ survival

Human HIP iPSCs were differentiated into HIP iECs. Using lentiviral particles, plasmids carrying the SERPINA1 cDNA were introduced into HIP iECs for the generation of HIP iEC$_{SERPINA1}$. A clone with high SERPINA1 expression was picked for XCelligence assays. **Figure 29** shows that HIP iEC$_{SERPINA1}$ were not killed after incubation with unstimulated PMNs but did so when the PMNs were stimulated with either sonicated target cells or IL-2. This confirms that cell products are at risk of PMN killing after transplantation when cells die and release DAMPs or trigger some inflammatory environment.

Four different PMN inhibitory strategies were tested for their efficacy to prevent target cell killing by activated PMNs. The regimens CpG-52364 + colchicine and TAK-242 + BAY 85-8501 (**Figure 30**) as well as CpG-52364 + BAY 85-8501 and Idelalisib + Apocynine + colchicine (**Figure 31**) showed that PMN killing was effectively inhibited.

### Methods

**Human HIP iPSC differentiation into HIP iECs:** Human gene edited HIP iPSC were plated on diluted Matrigel (356231, Corning) in 6-well plates and maintained in Essential 8 Flex media (Thermo Fisher Scientific, Carlsbad, CA). The differentiation was started at 60% confluency, and media was changed to RPMI-1640 containing 2% B-27 minus insulin (both Gibco) and 5 μM CHIR-99021 (Selleckchem). On day 2, the media was changed to reduced media: RPMI-1640 containing 2% B-27 minus insulin (Gibco) and 2 μM CHIR-99021 (Selleckchem). From day 4 to day 7, cells were exposed to RPMI-1640 EC media, RPMI-1640 containing 2% B-27 minus insulin plus 50 ng/mL human vascular endothelial growth factor (VEGF; R&D Systems), 10 ng/mL human fibroblast growth factor basic (FGFb; R&D Systems), 10 μM Y-27632 (Sigma-Aldrich), and 1 μM SB 431542 (Sigma-Aldrich). Endothelial cell clusters were visible from day 7 and cells were maintained in EGM-2 SingleQuots media (Lonza) plus 10% FCS hi (Gibco), 25 ng/mL VEGF, 2 ng/mL FGFb, 10 μM Y-27632 (Sigma-Aldrich), and 1 μM SB 431542 (Sigma-Aldrich). The differentiation process was completed after 14 days und undifferentiated cells detached during the differentiation process. For purification, cells were treated with 20 μM PluriSln-1 (StemCell Technologies, Vancouver, BC, Canada) for 48 h. The highly purified HIP iECs were cultured in EGM-2 SingleQuots media (Lonza) plus supplements and 10% FCS hi (Gibco). TrypLE Express was used for passaging the cells 1:3 every 3 to 4 days.

**Lentiviral overexpression of SERPINA1:** HIP iECs were transduced with lentiviral particles carrying a plasmid with the Serpina1 cDNA (RC202082L2V, Origene, Rockville, MD). The pool of cells was expanded and clones were picked and clonally expanded. The clone with the highest SERPINA1 mRNA expression was chosen.

IX. Exemplary sequences:

**SEQ ID NO:1 - Human B-2-Microglobulin**

```
MSRSVALAVLALLSLSGLEAIQRTPKIQVYSRHPAENGKSNFLNCYVSGFHPSDIEVDLLKN
GERIEKVEHSDLSFSKDWSFYLLYYTEFTPTEKDEYACRVNHVTLSQPKIVKWDRDI
```

**SEQ ID NO:2 - Human CIITA protein, 160 amino acid N-terminus**

```
MRCLAPRPAGSYLSEPQGSSQCATMELGPLEGGYLELLNSDADPLCLYHFYDQMDLAGEEEI
ELYSEPDTDTINCDQFSRLLCDMEGDEETREAYANIAELDQYVFQDSQLEGLSKDIFKHIGP
DEVIGESMEMPAEVGQKSQKRPFPEELPADLKHWKP
```

**SEQ ID NO:3 - Human CD47**

```
MWPLVAALLLGSACCGSAQLLFNKTKSVEFTFCNDTVVIPCFVTNMEAQNTTEVYVKWKFKG

RDIYTFDGALNKSTVPTDFSSAKIEVSQLLKGDASLKMDKSDAVSHTGNYTCEVTELTREGE
TIIELKYRVVSWFSPNENILIVIFPIFAILLFWGQFGIKTLKYRSGGMDEKTIALLVAGLVI
TVIVIVGAILFVPGEYSLKNATGLGLIVTSTGILILLHYYVFSTAIGLTSFVIAILVIQVIA
YILAVVGLSLCIAACIPMHGPLLISGLSILALAQLLGLVYMKFVE
```

**SEQ ID NO:4 - Herpes Simplex Virus Thimidine Kinase (HSV-tk)**

```
MASYPCHQHASAFDQAARSRGHSNRRTALRPRRQQEATEVRLEQKMPTLLRVYIDGPHGMGK
TTTTQLLVALGSRDDIVYVPEPMTYWQVLGASETIANIYTTQHRLDQGEISAGDAAVVMTSA
QITMGMPYAVTDAVLAPHVGGEAGSSHAPPPALTLIFDRHPIAALLCYPAARYLMGSMTPQA
VLAFVALIPPTLPGTNIVLGALPEDRHIDRLAKRQRPGERLDLAMLAAIRRVYGLLANTVRY
LQGGGSWWEDWGQLSGTAVPPQGAEPQSNAGPRPHIGDTLFTLFRAPELLAPNGDLYNVFAW
ALDVLAKRLRPMHVFILDYDQSPAGCRDALLQLTSGMVQTHVTTPGSIPTICDLARTFAREM
GEAN
```

**SEQ ID NO:5 - *Escherichia coli* Cytosine Deaminase (EC-CD)**

MSNNALQTIINARLPGEEGLWQIHLQDGKISAIDAQSGVMPITENSLDAEQGLVIPPFVEPH
IHLDTTQTAGQPNWQSGTLFEGIERWAERKALLTHDDVKQRAWQTLKWQIANGIQHVRTHV
DVSDATLTALKAMLEVKQEVAPWIDLQIVAFPQEGILSYPNGEALLEEALRLGADVVGAIPH
FEFTREYGVESLHKTFALAQKYDRLIDVHCDEIDDEQSRFVETVAALAHHEGMGARVTASHT
TAMHSYNGAYTSRLFRLLKMSGINFVANPLVNIHLQGRFDTYPKRRGITRVKEMLESGINVC
FGHDDVFDPWYPLGTANMLQVLHMGLHVCQLMGYGQINDGLNLITHHSARTLNLQDYGIAAG
NSANLIILPAENGFDALRRQVPVRYSVRGGKVIASTQPAQTTVYLEQPEAIDYKR

**SEQ ID NO:6 - Truncated human Caspase 9**

GFGDVGALESLRGNADLAYILSMEPCGHCLIINNVNFCRESGLRTRTGSNIDCEKLRRRFSS
LHFMVEVKGDLTAKKMVLALLELAQQDHGALDCCVVVILSHGCQASHLQFPGAVYGTDGCPV
SVEKIVNIFNGTSCPSLGGKPKLFFIQACGGEQKDHGFEVASTSPEDESPGSNPEPDATPFQ
EGLRTFDQLDAISSLPTPSDIFVSYSTFPGFVSWRDPKSGSWYVETLDDIFEQWAHSEDLQS
LLLRVANAVSVKGIYKQMPGCFNFLRKKLFFKTS

**SEQ ID NO:7 - Human CD64**
**NM 000566**

MWFLTTLLLWVPVDGQVDTTKAVITLQPPWVSVFQEETVTLHCEVLHLPGSSSTQWFLNGTA
TQTSTPSYRITSASVNDSGEYRCQRGLSGRSDPIQLEIHRGWLLLQVSSRVFTEGEPLALRC
HAWKDKLVYNVLYYRNGKAFKFFHWNSNLTILKTNISHNGTYHCSGMGKHRYTSAGISVTVK
ELFPAPVLNASVTSPLLEGNLVTLSCETKLLLQRPGLQLYFSFYMGSKTLRGRNTSSEYQIL
TARREDSGLYWCEAATEDGNVLKRSPELELQVLGLQLPTPVWFHVLFYLAVGIMFLVNTVLW
VTIRKELKRKKKWDLEISLDSGHEKKVISSLQEDRHLEEELKCQEQKEEQLQEGVHRKEPQG
AT

**SEQ ID NO:8 - Human CD52**
**NM _001803**
MKRFLFLLLTISLLVMVQIQTGLSGQNDTSQTSSPSASSNISGGIFLFFVANAIIHLFCFS

**SEQ ID NO:9 - Human CD16 FCGR3A**
**NM_001803**

MAEGTLWQILCVSSDAQPQTFEGVKGADPPTLPPGSFLPGPVLWWGSLARLQTEKSDEVSRK
GNWWVTEMGGGAGERLFTSSCLVGLVPLGLRISLVTCPLQCGIMWQLLLPTALLLLVSAGMR
TEDLPKAVVFLEPQWYRVLEKDSVTLKCQGAYSPEDNSTQWFHNESLISSQASSYFIDAATV
DDSGEYRCQTNLSTLSDPVQLEVHIGWLLLQAPRWVFKEEDPIHLRCHSWKNTALHKVTYLQ

NGKGRKYFHHNSDFYIPKATLKDSGSYFCRGLFGSKNVSSETVNITITQGLAVSTISSFFPP
GYQVSFCLVMVLLFAVDTGLYFSVKTNIRSSTRDWKDHKFKWRKDPQDK

**SEQ ID NO:10 - Human CD16 FCGR3B**
**NM_001803**

MWQLLLPTALLLLVSAGMRTEDLPKAVVFLEPQWYSVLEKDSVTLKCQGAYSPEDNSTQWFH
NENLISSQASSYFIDAATVNDSGEYRCQTNLSTLSDPVQLEVHIGWLLLQAPRWVFKEEDPI
HLRCHSWKNTALHKVTYLQNGKDRKYFHHNSDFHIPKATLKDSGSYFCRGLVGSKNVSSETV
NITITQGLAVSTISSFSPPGYQVSFCLVMVLLFAVDTGLYFSVKTNI

**SEQ ID NO:11 - Human CD32 FCGR2A**
**NM_001803**

MTMETQMSQNVCPRNLWLLQPLTVLLLLASADSQAAAPPKAVLKLEPPWINVLQEDSVTLTC
QGARSPESDSIQWFHNGNLIPTHTQPSYRFKANNNDSGEYTCQTGQTSLSDPVHLTVLSEWL
VLQTPHLEFQEGETIMLRCHSWKDKPLVKVTFFQNGKSQKFSHLDPTFSIPQANHSHSGDYH
CTGNIGYTLFSSKPVTITVQVPSMGSSSPMGIIVAVVIATAVAAIVAAVVALIYCRKKRISA
NSTDPVKAAQFEPPGRQMIAIRKRQLEETNNDYETADGGYMTLNPRAPTDDDKNIYLTLPPN
DHVNSNN

**SEQ ID NO:12 - Human CD32 FCGR2B**
**NM_001803**

MGILSFLPVLATESDWADCKSPQPWGHMLLWTAVLFLAPVAGTPAAPPKAVLKLEPQWINVL
QEDSVTLTCRGTHSPESDSIQWFHNGNLIPTHTQPSYRFKANNNDSGEYTCQTGQTSLSDPV
HLTVLSEWLVLQTPHLEFQEGETIVLRCHSWKDKPLVKVTFFQNGKSKKFSRSDPNFSIPQA
NHSHSGDYHCTGNIGYTLYSSKPVTITVQAPSSSPMGIIVAVVTGIAVAAIVAAVVALIYCR
KKRISALPGYPECREMGETLPEKPANPTNPDEADKVGAENTITYSLLMHPDALEEPDDQNRI

**SEQ ID NO:13 - Human CD32 FCGR2C**
**NM_001803**

MGILSFLPVLATESDWADCKSPQPWGHMLLWTAVLFLAPVAGTPAAPPKAVLKLEPQWINVL
QEDSVTLTCRGTHSPESDSIPWFHNGNLIPTHTQPSYRFKANNNDSGEYTCQTGQTSLSDPV
HLTVLSEWLVLQTPHLEFQEGETIVLRCHSWKDKPLVKVTFFQNGKSKKFSRSDPNFSIPQA
NHSHSGDYHCTGNIGYTLYSSKPVTITVQAPSSSPMGIIVAVVTGIAVAAIVAAVVALIYCR
KKRISANSTDPVKAAQFEPPGRQMIAIRKRQPEETNNDYETADGGYMTLNPRAPTDDDKNIY
LTLPPNDHVNSNN

**SEQ ID NO:14 - Human CD99**
**NP_002405.1**

MARGAALALL LFGLLGVLVA APDGGFDLSD ALPDNENKKP TAIPKKPSAG
DDFDLGDAVV DGENDDPRPP NPPKPMPNPN PNHPSSSGSF SDADLADGVS
GGEGKGGSDG GGSHRKEGEE ADAPGVIPGI VGAVVVAVAG AISSFIAYQK
KKLCFKENAE QGEVDMESHR NANAEPAVQR TLLEK

**SEQ ID NO:15 - Human CD200**
**NP_001352780.1**

MERLVIRMPF SHLSTYSLVW VMAAVVLCTA QVQVVTQDER EQLYTPASLK
CSLQNAQEAL IVTWQKKKAV SPENMVTFSE NHGVVIQPAY KDKINITQLG
LQNSTITFWN ITLEDEGCYM CLFNTFGFGK ISGTACLTVY VQPIVSLHYK
FSEDHLNITC SATARPAPMV FWKVPRSGIENSTVTLSHPN GTTSVTSILH

IKDPKNQVGK EVICQVLHLG TVTDFKQTVN KGYWFSVPLLLSIVSLVILL
VLISILLYWK RHRNQDRGEL SQGVQKMT

**SEQ ID NO:16 - Murine Alpha-1 Antitrypsin (A1AT)**

```
>NP_033273.1 alpha-1-antitrypsin 1-5 precursor [Mus musculus]
MTPSISWCLLLLAGLCCLVPSFLAEDVQETDTSQKDQSPASHEIATNLGDFAISLYRELVHQ
SNTSNIFFSPVSIATAFAMLSLGSKGDTHTQILEGLQFNLTQTSEADIHNSFQHLLQTLNRP
DSELQLSTGNGLFVNNDLKLVEKFLEEAKNHYQAEVFSVNFAESEEAKKVINDFVEKGTQGK
IVEAVKKLEQDTVFVLANYILFKGKWKKPFDPENTKQAEFHVDESTTVKVPMMTLSGMLDVH
HCSTLSSWVLLMDYAGNATAVFLLPDDGKMQHLEQTLNKELISKFLLNRRRRLAQIHIPRLS
ISGNYNLETLMSPLGITRIFNSGADLSGITEENAPLKLSQAVHKAVLTIDETGTEAAAATVL
QGGFLSMPPILHFNRPFLFIIFEEHSQSPLFVGKVVDPTHK
```

**SEQ ID NO:17 - Human Alpha-1 Antitrypsin (A1AT)**

```
>NP_001002236.1 alpha-1-antitrypsin precursor [Homo sapiens]
MPSSVSWGILLLAGLCCLVPVSLAEDPQGDAAQKTDTSHHDQDHPTFNKITPNLAEFAFSLYRQLAHQ
SNSTNIFFSPVSIATAFAMLSLGTKADTHDEILEGLNFNLTEIPEAQIHEGFQELLRTLNQPDSQLQL
TTGNGLFLSEGLKLVDKFLEDVKKLYHSEAFTVNFGDTEEAKKQINDYVEKGTQGKIVDLVKELDRDT
VFALVNYIFFKGKWERPFEVKDTEEEDFHVDQVTTVKVPMMKRLGMFNIQHCKKLSSWVLLMKYLGNA
TAIFFLPDEGKLQHLENELTHDIITKFLENEDRRSASLHPKLSITGTYDLKSVLGQLGITKVFSNGA
DLSGVTEEAPLKLSKAVHKAVLTIDEKGTEAAGAMFLEAIPMSIPPEVKFNKPFVFLMIEQNTKSPLF
MGKVVNPTQK
```

All publications and patent documents disclosed or referred to herein are incorporated by reference in their entirety. The foregoing description has been presented only for purposes of illustration and description. This description is not intended to limit the invention to the precise form disclosed. It is intended that the scope of the invention be defined by the claims appended hereto.

Aspects and features of the present disclosure are set out in the following numbered paragraphs:

1. A method of inhibiting polymorphonuclear (PMN) cells from killing a cell transplanted into a subject, comprising administering a PMN-inhibitory agent into said subject.

2. The method of paragraph 1, wherein said PMN inhibitory agent is an inflammatory inhibitor, a danger-associated molecular pattern (DAMP) receptor inhibitor, a DAMP pathway inhibitor, a secretory enzyme release inhibitor, a reactive oxygen pathway inhibitor, a microtubule polymerization inhibitor, or a combination thereof.

3. The method of either one of paragraphs 1 or 2, wherein said PMN-inhibitory agent is administered simultaneously with said transplanted cells or prior to said cell transplantation.

4. The method of any one of paragraphs 1 to 3, wherein said PMN-inhitibory agent inhibits an interleukin 2 (IL-2), IL-2 receptor (IL-2R), toll-like receptor 4 (TLR4), TLR7, TLR8, TLR9, a toll-like receptor pathway, a neutrophil elastase (NE) receptor, an NADPH oxidase, microtubule polymerization, or wherein said PMN-inhitibory agent stimulates the cluster of differentiation 200 receptor (CD200R), PIRLα, or SIRL-1.

5. The method of any one of paragraphs 1 to 4, wherein said PMN-inhibitory agent is selected from the group consisting of cyclosporin A (CsA), dexamethasone, an anti-SIRL-1 antibody, a CD200 chimera, a CD200 expressed on cells, a CD99 chimera, a CD99 expressed on cells, BAY 85-8501, alpha-1 antitrypsin (A1AT), TAK-242, apocynin, idelalisib, CpG-52364, colchicine, and the anti-TLR4 antibody NI-0101.

6. The method of paragraph 5, wherein said method comprises the combination of an anti-SIRL-1 antibody and BAY 85-8501.

7. The method of paragraph 5, wherein said method comprises the combination of CsA and BAY 85-8501.

8. The method of any one of paragraphs 1 to 7, wherein said method further comprises administering Colcicine, Idelalisib, or oligonucleotide CpG-52364.

9. The method of either one of paragraphs 1 or 2, wherein said PMN-inhibitory agent is expressed on the surface of said transplanted cells.

10. The method of paragraph 9, wherein said PMN-inhibitory agent is CD200 or CD99.

11. The method of any one of paragraphs 1-10, wherein said transplanted cell is derived from a hypoimmune pluripotent (HIP) cell, a HIP cell that is AB blood group O and Rhesus Factor negative (HIPO-), an induced pluripotent stem cell (iPSC), an embryonic stem cell (ESC), or a derivative thereof.

12. The method of any one of paragraphs 1-11, wherein said transplanted cell is selected from the group consisting of a chimeric antigen receptor (CAR) cell, an endothelial cell, a dopaminergic neuron, a pancreatic islet cell, a cardiomyocyte, and a retinal pigment endothelium cell.

13. The method of any one of paragraphs 1-12, wherein said transplanted cell is from a species selected from the group consisting of a human, monkey, cow, pig, chicken, turkey, horse, sheep, goat, donkey, mule, duck, goose, buffalo, camel, yak, llama, alpaca, mouse, rat, dog, cat, hamster, and guinea pig.

14. The method of paragraph 13, wherein said transplanted cell is human.

15. A pharmaceutical composition, comprising a cell derived from a pluripotent cell or a derivative thereof, a PMN-inhibitory agent, and a pharmaceutical excipient.

16. The pharmaceutical composition of paragraph 15, wherein said PMN inhibitory agent is an inflammatory inhibitor, a danger-associated molecular pattern (DAMP) inhibitor, a DAMP pathway inhibitor, or a combination thereof.

17. The pharmaceutical composition of either one of paragraphs 15 or 16, wherein said PMN-inhibitory agent and said cell are delivered to a subject using separate delivery methods.

18. The pharmaceutical composition of any one of paragraphs 15 to 17, wherein said PMN-inhitibory agent inhibits an interleukin 2 (IL-2) receptor (IL-2R), toll-like receptor 4 (TLR4), TLR7, TLR8, TLR9 or any of its downstream targets, neutrophil elastase (NE), NADPH-oxidase for superoxide production, or engages cluster of differentiation 200 receptor (CD200R), PIRLα, or SIRL-1.

19. The pharmaceutical composition of any one of paragraphs 15 to 18, wherein said PMN-inhibitory agent is selected from the group consisting of cyclosporin A (CsA), dexamethasone, an anti-SIRL-1 antibody, a CD200 chimera, CD200 expressed on cells, a CD99 chimera, CD99 expressed on cells, BAY 85-8501, alpha-1 antitrypsin (A1AT), TAK-242, apocynin, idelalisib, CpG-52364, colchicine, or the anti-TLR4 antibody NI-0101.

20. The pharmaceutical composition of paragraph 19, wherein said PMN-inhibitory agent comprises the combination of an anti-SIRL-1 antibody and BAY 85-8501.

21. The pharmaceutical composition of paragraph 19, further comprising the combination of CsA and BAY 85-8501.

22. The pharmaceutical composition of any one of paragraphs 15 to 21, further comprising Colchicine, Idelalisib, or oligonucleotide CpG-52364.

23. The pharmaceutical composition of either one of paragraphs 15 or 16, wherein said PMN-inhibitory agent is expressed on the surface of said cell.

24. The pharmaceutical composition of paragraph 23, wherein said PMN-inhibitory agent is CD200 or CD99.

25. The pharmaceutical composition of any one of paragraphs 15-24, wherein said cell is derived from a hypoimmune pluripotent (HIP) cell, a HIP cell that is AB blood group O, a Rhesus Factor negative (HIPO-), an induced pluripotent stem cell (iPSC), or an embryonic stem cell (ESC).

26. The pharmaceutical composition of any one of paragraphs 15-25, wherein said cell is selected from the group consisting of a chimeric antigen receptor (CAR) cell, an endothelial cell, a dopaminergic neuron, a pancreatic islet cell, a cardiomyocyte, and a retinal pigment endothelium cell.

27. The pharmaceutical composition of any one of paragraphs 15-26, wherein said transplanted cell is from a species selected from the group consisting of a human, monkey, cow, pig, chicken, turkey, horse, sheep, goat, donkey, mule, duck, goose, buffalo, camel, yak, llama, alpaca, mouse, rat, dog, cat, hamster, and guinea pig.

28. The pharmaceutical composition of paragraph 27, wherein said transplanted cell is human.

29. A medicament for treating a disease, comprising a cell derived from a pluripotent cell or a derivative thereof, a PMN-inhibitory agent, and a pharmaceutical excipient.

30. The medicament of paragraph 29, wherein said derivative cell is selected from the group consisting of a chimeric antigen receptor (CAR) cell, an endothelial cell, a dopaminergic neuron, a pancreatic islet cell, a cardiomyocyte, and a retinal pigment endothelium cell.

31. The medicament of paragraph 29, wherein said disease is selected from the group consisting of Type I Diabetes, a cardiac disease, a neurological disease, a cancer, an ocular disease, and a vascular disease.

32. A human hypo-immunogenic pluripotent (HIP) cell or a derivative thereof comprising:

   a. an overexpressed alpha-1 antitrypsin (A1AT) protein relative to a parent HIP cell;
   b. an endogenous Major Histocompatibility Antigen Class I (HLA-I) function that is reduced when compared to a parent pluripotent cell;
   c. an endogenous Major Histocompatibility Antigen Class II (HLA-II) function that is reduced when compared to said parent pluripotent cell;
   d. an increased CD47 function that reduces susceptibility to NK cell killing;
   e. an ABO blood group type O (O); and
   f. a Rhesus Factor (Rh) blood type negative (-)

wherein said human hypo-immunogenic pluripotent O- (HIPO-) cell is less susceptible to rejection when transplanted into a subject when compared with an otherwise similar hypo-immunogenic pluripotent (HIP) cell that is an ABO blood group or Rh factor mismatch to said subject.

33. The HIPO- cell of paragraph 32, wherein said A1AT protein comprises at least a 90% sequence identity to SEQ ID NO:17.

34. The HIPO- cell of paragraph 33, wherein said A1AT protein comprises the the sequence of SEQ ID NO:17.

35. The HIPO- cell of any one of paragraphs 32-34, further comprising an elevated level of CD16, CD32, or CD64 protein expression when compared to a parent HIPO- cell, wherein said elevated protein expression causes said modified pluripotent cell to be less susceptible to antibody dependent cellular cytoxicity (ADCC) or complement-dependent cytotoxicity (CDC).

36. A differentiated cell derived from either one of the HIPO- cells of paragraphs 32-35, wherein said differentiated cell is an endothelial cell or a fibroblast cell.

37. The differentiated cell of paragraph 36, wherein said differentiated cell is an endothelial cell.

38. The differentiated cell of paragraph 36, wherein said differentiated cell is a fibroblast cell.

39. A pharmaceutical composition, comprising the differentiated cell of any one of paragraphs 36-38, an additional PMN-inhibitory agent, and a pharmaceutical excipient.

40. The pharmaceutical composition of paragraph 39, wherein said additional PMN inhibitory agent is an inflammatory inhibitor, a danger-associated molecular pattern (DAMP) inhibitor, or a combination thereof.

41. The pharmaceutical composition of either one of either one of paragraphs 39 or 40, wherein said additional PMN-inhibitory agent and said cell are delivered to a subject using separate delivery methods.

42. The pharmaceutical composition of any one of paragraphs 39-41, wherein said PMN-inhitibory agent inhibits an interleukin 2 (IL-2) receptor (IL-2R), toll-like receptor 4 (TLR4), TLR7, TLR8, TLR9 or any of its downstream targets, neutrophil elastase (NE), NADPH-oxidase for superoxide production, or engages cluster of differentiation 200 receptor (CD200R), PIRL$\alpha$, or SIRL-1.

43. The pharmaceutical composition of any one of paragraphs 39-42, wherein said PMN-inhibitory agent is selected from the group consisting of cyclosporin A (CsA), dexamethasone, an anti-SIRL-1 antibody, a CD200 chimera, a CD99 chimera, BAY 85-8501, TAK-242, apocynin, idelalisib, CpG-52364, colchicine, or the anti-TLR4 antibody NI-0101.

44. A method of treating an alpha-1 antitrypsin deficiency (A1AD) in a subject comprising administering the pharmaceutical composition of any one of paragraphs 39-43 to said subject.

**Claims**

1. A modified therapeutic cell, comprising an increased CD200 receptor (CD200R) ligand and CD99 receptor (CD99R) ligand when compared to a parental cell that has not been modified, wherein said modified therapeutic cell comprises:

   a. a reduced or eliminated cell-surface Major Histocompatibility Antigen Class I (HLA-I) when compared to said parental cell that has not been modified;
   b. a reduced or eliminated cell-surface Major Histocompatibility Antigen Class II (HLA-II) when compared to said parental cell that has not been modified; and
   c. an increased CD47 expression from a transgene or from a modified chromosomal expression sequence when compared to said parental cell that has not been modified;

   wherein said CD200R ligand is a polymorphonuclear cell (PMN) inhibitor.

2. The modified therapeutic cell of claim 1, wherein said CD200R ligand has at least a 90% sequence identity with SEQ ID NO: 15.

3. The modified therapeutic cell of claim 2, wherein said CD200R ligand has the sequence of SEQ ID NO:15.

4. The modified therapeutic cell of any one of claims 1-3, wherein said CD99R ligand has at least a 90% sequence identity with SEQ ID NO: 14.

5. The modified therapeutic cell of claim 4, wherein said CD99R ligand has the sequence of SEQ ID NO:14.

6. The modified therapeutic cell of any one of claims 1-5, wherein said modified therapeutic cell is ABO blood group type O and Rhesus factor negative (Rh-).

7. The modified therapeutic cell of any one of claims 1-6, wherein said modified therapeutic cell is a chimeric antigen receptor (CAR) cell, an endothelial cell, a dopaminergic neuron, a pancreatic islet cell, a cardiomyocyte, or a retinal pigment endothelium cell.

8. The modified therapeutic cell of any one of claims 1-7, wherein said cell is derived or differentiated from a hypoimmune cell, a hypoimmune pluripotent (HIP) cell, a hypoimmune or HIP cell that is AB blood group O, a Rhesus Factor negative (Rh-) cell, a hypoimmune or HIP cell that is blood group O and Rh-, an induced pluripotent stem cell (iPSC), or an embryonic stem cell (ESC).

9. The modified therapeutic cell of any one of claims 1-8, further comprising a CD64 molecule on its cell surface.

10. The modified therapeutic cell of any one of claims 1-9 for use in treating a disease.

11. The modified therapeutic cell for use of claim 10, wherein said disease is diabetes, a thyroid disease, a cardiac disease, a neurological disease, a cancer, an ocular disease, or a vascular disease.

12. A pharmaceutical composition, comprising the modified therapeutic cell of any one of claims 1-9 and a pharmaceutical excipient.

13. The pharmaceutical composition of claim 12, further comprising a second PMN-inhibitory agent.

14. The pharmaceutical composition of claim 13, wherein said second PMN-inhibitory agent is an inflammatory inhibitor, a Danger-Associated Molecular Pattern (DAMP) inhibitor, a DAMP pathway inhibitor, or a combination thereof.

15. The pharmaceutical composition of claim 13, wherein said second PMN-inhibitory agent inhibits toll-like receptor 4 (TLR4), TLR9, a TLR9 downstream target, or neutrophil elastase (NE).

16. The pharmaceutical composition of claim 13, wherein said second PMN-inhibitory agent is TAK-242, CpG-52364, or BAY 85-8501.

Figure 1

Figure 2

Figure 3

Figure 4

FIG. 5A

FIG. 5B

FIG. 6

Figure 7

FIG. 8

FIG. 9A

FIG. 9B

Figure 10

Figure 11

FIG. 12

Figure 13

Figure 14

Figure 15

Figure 16

Figure 17

Figure 18A

Figure 18B

Figure 19

Figure 20A

Figure 20B

Figure 21A

Figure 21B

Figure 22

Figure 23

Figure 24

Figure 25

Serpina-/- LPS
no cell injection

Figure 26

Figure 27

Figure 28

Figure 29

PMN killing

Figure 30

Figure
31

PMN killing with PMN inhibitors

PMN killing with PMN inhibitors

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62961636 A
- US 63003264 A
- WO 2018132783 A
- US 62846399
- US 62855499
- US 62915601
- US 8796443 B
- WO 9715664 A
- GB 2211504 A
- WO 2011146862 A
- US 4427777 A
- US 5606042 A
- US 5633130 A

- US 5731426 A
- US 6184017 B
- US 4609627 A
- WO 9923210 A
- US 1942123 W
- US 1942117 W
- WO 62698973 A
- WO 62698978 A
- WO 62698981 A
- WO 62698984 A
- WO 62846399 A
- WO 62855499 A
- WO 62915601 A

**Non-patent literature cited in the description**

- **COWAN, C. A.** *New England J. Med.*, 2004, vol. 350, 13
- **ZHOU et al.** *Stem Cells*, 2009, vol. 27 (11), 2667-74
- **HUANGFU et al.** *Nature Biotechnol.*, 2008, vol. 26 (7), 795
- **WOLTJEN et al.** *Nature*, 2009, vol. 458 (7239), 766-770
- **ZHOU et al.** *Cell Stem Cell*, 2009, vol. 8, 381-384
- **RYAN, A. K.** ; **ROSENFELD, M. G.** *Genes Dev.*, 1997, vol. 11, 1207-1225
- **DANG, D. T.** ; **PEVSNER, J.** ; **YANG, V. W.** *Cell Biol.*, 2000, vol. 32, 1103-1121
- **NAKAGAWA et al.** *Nature Biotechnology*, 2007, vol. 26, 101-106
- **ADHIKARY, S.** ; **EILERS, M.** *Nat. Rev. Mol. Cell Biol.*, 2005, vol. 6, 635-645
- **DANG, D. T. et al.** *Int. J. Biochem. Cell Biol.*, 2000, vol. 32, 1103-1121
- **NEEDLEMAN** ; **WUNSCH.** *J. Mol. Biol.*, 1970, vol. 48, 443
- **PEARSON** ; **LIPMAN.** *Proc. Nat'l. Acad. Sci. USA*, 1988, vol. 85, 2444
- **ALTSCHUL et al.** *J. Mol. Biol.*, 1990, vol. 215, 403-410
- **AUSUBEL et al.** Current Protocols in Molecular Biology. Wiley Interscience Publishers, 1995
- **FIERS et al.** *Nature*, 1978, vol. 273, 113-120
- **GREENAWAY, P. J. et al.** *Gene*, 1982, vol. 18, 355-360

- **TAKAHASHI** ; **YAMANAKA.** *Cell*, 2006, vol. 126, 663-676
- **SEKI et al.** *World J. Stem Cells*, 2015, vol. 7 (1), 116-125
- Methods in Molecular Biology: Pluripotent Stem Cells. Methods and Protocols. Springer, 2013
- **DIECKE et al.** *Sci Rep.*, 28 January 2015, vol. 5, 8081
- **BURRIDGE et al.** *PLoS One*, 2011, vol. 6 (4), 18293
- **GORNALUSSE et al.** *Nature Biotech.*
- **BARESE et al.** *Mol. Therap.*, 2012, vol. 20 (10), 1932-1943
- **XU et al.** *Cell Res.*, 1998, vol. 8, 73-8
- **STASI et al.** *N. Engl. J. Med*, 2011, vol. 365, 18
- **TEY et al.** *Biol. Blood Marrow Transplant.*, 2007, vol. 13, 913-924
- **OLSSON et al.** *Transfusion Clinique et Biologique*, 2004, vol. 11, 33-39
- **PETTINATO** ; **SNYKERS et al.** *Methods Mol Biol*, 2011, vol. 698, 305-314
- **SI-TAYEB et al.** *Hepatology*, 2010, vol. 51, 297-305
- **ASGARI et al.** *Stem Cell Rev*, 2013, 493-504
- **KAMAO et al.** *Stem Cell Reports*, 2014, vol. 2, 205-18
- **MATZINGER.** *Ann. Rev. Immunol.*, 1994, vol. 12, 991-1045
- **STEEVELS TA.** *Eur J Immunol.*, 2013, vol. 43 (5), 1297-308
- **VON NUSSBAUM F.** *Chem. Med. Chem.*, 2015, vol. 10, 1163-73

- **WATZ H.** *Pulm. Pharmacol. Ther.*, 2019, vol. 56, 86-93
- **POLVERINO E.** *Chest.*, 2017, vol. 152 (2), 249-262
- **STEFANSKA J.** *Mediators Inflamm.*, 2008, 106507
- **BOREL et al.** *Proc Natl Acad Sci USA*, 2018, vol. 115, 2788-2793